(19) **Europäisches Patentamt / European Patent Office / Office européen des brevets**

(11) **EP 2 194 028 B1**

(12) **EUROPEAN PATENT SPECIFICATION**

(45) Date of publication and mention of the grant of the patent:
**26.01.2022 Bulletin 2022/04**

(21) Application number: **10151585.6**

(22) Date of filing: **08.08.2003**

(51) International Patent Classification (IPC):
**C01F 17/247** (2020.01)  **C01F 17/271** (2020.01)
**C02F 1/28** (2006.01)  **C02F 1/52** (2006.01)
**C09C 1/36** (2006.01)  **A61K 33/244** (2019.01)
**B01J 20/02** (2006.01)  **B01J 20/06** (2006.01)
**B01J 20/28** (2006.01)  **B01J 20/30** (2006.01)
**B01J 20/32** (2006.01)  **A61P 1/00** (2006.01)
**A61P 3/00** (2006.01)  **A61P 3/12** (2006.01)
**A61P 7/08** (2006.01)  **A61P 43/00** (2006.01)

(52) Cooperative Patent Classification (CPC):
**C02F 1/281; A61K 33/24; A61K 33/244;
A61P 1/00; A61P 3/00; A61P 3/12; A61P 7/08;
A61P 43/00; B01J 20/0207; B01J 20/0277;
B01J 20/0288; B01J 20/06; B01J 20/28057;
B01J 20/3078; B01J 20/3234;** (Cont.)

(54) **Rare earth metal compounds, methods of making, and methods of using the same**

Seltenerdmetallzusammensetzungen, Verfahren zu deren Herstellung und Verwendung

Composés de métaux de terres rares, procédés de fabrication et leurs procédés d'utilisation

(84) Designated Contracting States:
**AT BE BG CH CY CZ DE DK EE ES FI FR GB GR HU IE IT LI LU MC NL PT RO SE SI SK TR**

(30) Priority: **14.08.2002 US 403868 P**
**02.12.2002 US 430284**
**08.04.2003 US 461175**
**23.05.2003 US 444774**

(43) Date of publication of application:
**09.06.2010 Bulletin 2010/23**

(62) Document number(s) of the earlier application(s) in accordance with Art. 76 EPC:
**03749033.1 / 1 529 015**

(73) Proprietor: **Unicycive Therapeutics, Inc.**
**Los Altos, CA 94022 (US)**

(72) Inventors:
• **Moerck, Rudi E.**
**Sanford, FL 32771 (US)**
• **Spitler, Timothy M.**
**Fernley, NV 89408 (US)**
• **Schauer, Edward**
**Sparks, NV 89431 (US)**
• **Prochazka, Jan**
**Reno, NV 89503 (US)**

(74) Representative: **Bergenstråhle & Partners AB**
**P.O. Box 17704**
**118 93 Stockholm (SE)**

(56) References cited:
**WO-A-02/22258**    **AU-B2- 601 998**
**US-A- 3 692 671**    **US-A- 4 497 785**
**US-A- 4 919 902**    **US-A- 4 929 787**
**US-A- 5 968 976**    **US-A- 6 146 539**
**US-A1- 2002 051 822**

• **DATABASE CHEMABS [Online] CHEMICAL ABSTRACTS SERVICE, COLUMBUS, OHIO, US; 1 January 2000 (2000-01-01), OLAFSEN, ANJA ET AL: "Synthesis of rare earth oxide carbonates and thermal stability of Nd2O2CO3 II" XP002280167 retrieved from STN Database accession no. 132:18170 & JOURNAL OF MATERIALS CHEMISTRY , 9(10), 2697-2702 CODEN: JMACEP; ISSN: 0959-9428, 1999,**
• **LIU S ET AL: "Synthesis and structure characterization of hydrated lanthanum carbonate", HUANAN LIGONG DAXUE XUEBAO - JOURNAL OF SOUTH CHINA UNIVERSITY OF TECHNOLOGY, GUANGZHOU, CH, vol. 25, no. 10, 1 October 1997 (1997-10-01), pages 23-26, XP008116865, ISSN: 1000-565X**

- K. NAGASHIMA, H. WAKITA, A. MOCHIZUKI: "The synthesis of crystalline rare earth carbonates", BULLETIN OF THE CHEMICAL SOCIETY OF JAPAN, vol. 46, no. 1, 1973, pages 152-156,
- HUTCHISON A J: "CALCITRIOL, LANTHANUM CARBONATE, AND OTHER NEW PHOSPHATE BINDERS IN THE MANAGEMENT OF RENAL OSTEODYSTROPHY", PERITONEAL DIALYSIS INTERNATIONAL, PERGAMON PRESS, NEW YORK, NY, US, vol. 19, no. SUPPL. 02, 1 January 1999 (1999-01-01), pages S408-S412, XP009030319, ISSN: 0896-8608
- GRAFF L ET AL: "A POSSIBLE NON-ALUMINUM ORAL PHOSPHATE BINDER? ÖA COMPARATIVE STUDY ON DIETARY PHOSPHORUS ABSORPTION", RESEARCH COMMUNICATIONS IN CHEMICAL PATHOLOGY AND PHARMACOLOGY, PJD PUBLICATIONS LTD., WESTBURY, NY, US, vol. 89, no. 3, 1 September 1995 (1995-09-01), pages 373-388, XP000578701, ISSN: 0034-5164
- Y. WATANABE, S. MIYAZAKI, T. MARUYAMA, Y. SAITO: "Dissociation pressure of lanthanum dioxide carbonate", JOURNAL OF MATERIAL SCIENCE LETTERS, vol. 5, no. 2, February 1986 (1986-02), pages 135-136,
- A. OLAFSEN, H. FJELLVAG, S. STOLEN: "Heat capacities and entropies of La2O2CO3 from T= (12 to 300) K and of Nd2O2CO3 from T= (12 to 930) K, and their interpretation", JOURNAL OF CHEMICAL THERMODYNAMICS, vol. 31, no. 4, April 1999 (1999-04), pages 433-439,
- M. L. PANCHULA, M. AKINC: "Morphology of lanthanum carbonate particles prepared by homogeneous precipitation", JOURNAL OF THE EUROPEAN CERAMIC SOCIETY, vol. 16, no. 8, August 1996 (1996-08), pages 833-841,
- R. P. TURCOTTE, J. O. SAWYER, L. EYRING: "On the rare earth dioxymonocarbonates and their decomposition", INORGANIC CHEMISTRY, vol. 8, no. 2, 1 February 1969 (1969-02-01), pages 238-246,

(52) Cooperative Patent Classification (CPC): (Cont.)
**B01J 20/3236; C01F 17/247; C01F 17/271; C09C 1/3661;** C01P 2002/60; C01P 2002/72; C01P 2004/03; C01P 2004/61; C01P 2004/80; C01P 2006/12; C01P 2006/14; C01P 2006/90; C02F 1/001; C02F 2101/103; C02F 2101/105; C02F 2101/20; C02F 2103/026

**Description**

US 4,497,785 discloses a method for preparing high-density rare earth carbonate which comprises admixing, with agitation, an aqueous solution containing rare earth ions and an aqueous solution containing bicarbonate or carbonate ions, in the presence of added rare earth carbonate crystals, and separating rare earth carbonate from the aqueous solution.

US 4,929,787 discloses a process for the oxidative conversion of methane or natural gas to higher hydrocarbons, characterized in that a methane-oxygen mixture is passed over a contact mass, particularly rare-earth oxycarbonates, more particularly lanthanum oxycarbonate $La_2O_2(CO_3)$. US 4,919,902 discloses a catalytic composite for treating an exhaust gas comprising a support which is a refractory inorganic oxide having dispersed thereon lanthanum, at least one other rare earth component and at least one noble metal component selected from the group consisting of platinum, palladium, rhodium, ruthenium and iridium. An essential feature of said catalytic composite is that the lanthanum be present as crystalline particles of lanthanum oxide which have an average crystallite size of less than about 25 Å. An important feature in manufacturing the catalytic composite is the dispersion of lanthanum oxide onto said refractory inorganic oxide support. In a specific example, lanthanum may be dispersed on alumina as follows. A solution of a lanthanum salt is mixed with a hydrosol of aluminum, particles are formed from said lanthanum containing hydrosol, calcined to form an inorganic oxide particle containing lanthanum oxide, ground to give a powder of alumina containing finely dispersed lanthanum oxide.

Crystalline lanthanum carbonate was synthesized using ammonium bicarbonate as the precipitant. The X-ray diffraction data were indexed in the orthorhombic system with cell parameters a= 0.8487 nm, b= 0.9564 nm and c= 0. 4486 nm. The IR data for lanthanum carbonate showed the presence of two different carbonate groups. In the process of thermal decomposition of lanthanum carbonate to oxide, an intermediate $La_2O_3 \cdot CO_2$ phase was detected. LIU S ET AL: "Synthesis and structure characterization of hydrated lanthanum carbonate", HUANAN LIGONG DAXUE XUEBAO - JOURNAL OF SOUTH CHINA UNIVERSITY OF TECHNOLOGY, GUANGZHOU, CH, vol. 25, no. 10, 1 October 1997 (1997-10-01), pages 23-26, ISSN: 1000-565X.

The present invention relates to lanthanum compounds, particularly lanthanum compounds having a porous structure. The present invention also includes methods of making the porous lanthanum compounds and methods of using the compounds of the present invention. The compounds of the present invention can be used to bind or absorb metals such as arsenic, selenium, antimony and metal ions such as arsenic III+ and V+. The compounds of the present disclosure may therefore find use in water filters or other devices or methods to remove lanthanum and lanthanum ions from fluids, especially water.

The compounds of the present invention are also useful for binding or absorbing anions such as phosphate in the gastrointestinal tract of mammals. Accordingly, one use of the compounds of the present disclosure is to treat high serum phosphate levels in patients with end-stage renal disease undergoing kidney dialysis. In this aspect not covered by the claimed invention, the compounds may be provided in a filter that is fluidically connected with a kidney dialysis machine such that the phosphate content in the blood is reduced after passing through the filter.

In another aspect, the compounds can be used to deliver a lanthanum or other rare-earth metal compound that will bind phosphate present in the gut and prevent its transfer into the bloodstream. Compounds of the present invention can also be used to deliver drugs or to act as a filter or absorber in the gastrointestinal tract or in the blood stream. For example, the materials can be used to deliver inorganic chemicals in the gastrointestinal tract or elsewhere.

It has been found that the porous particle structure and the high surface area are beneficial to high absorption rates of anions. Advantageously, these properties permit the compounds of the present disclosure to be used to bind phosphate directly in a filtering device fluidically connected with kidney dialysis equipment.

The use of rare earth hydrated oxides, particularly hydrated oxides of La, Ce, and Y to bind phosphate is disclosed in Japanese published patent application 61-004529 (1986 ). Similarly, US Pat. No. 5,968,976 discloses a lanthanum carbonate hydrate to remove phosphate in the gastrointestinal tract and to treat hyperphosphatemia in patients with renal failure. It also shows that hydrated lanthanum carbonates with about 3 to 6 molecules of crystal water provide the highest removal rates. US Pat. No. 6,322,895 discloses a form of silicon with micron-sized or nano-sized pores that can be used to release drugs slowly in the body. US Pat. No. 5,782,792 discloses a method for the treatment of rheumatic arthritis where a "protein A immunoadsorbent" is placed on silica or another inert binder in a cartridge to physically remove antibodies from the bloodstream.

It has now unexpectedly been found that the specific surface area of compounds according to the present invention as measured by the BET method, varies depending on the method of preparation, and has a significant effect on the properties of the product. As a result, the specific properties of the resulting compound can be adjusted by varying one or more parameters in the method of making the compound. In this regard, the compounds of the present invention have a BET specific surface area of at least about 10 $m^2/g$ and may have a BET specific surface area of at least about 20 $m^2/g$ and alternatively may have a BET specific surface area of at least about 35 $m^2/g$. In one embodiment, the compounds have a BET specific surface area within the range of about 10 $m^2/g$ and about 40 $m^2/g$.

It has also been found that modifications in the preparation method of the lanthanum compounds will create different entities, e.g. different kinds of hydrated or amorphous oxycarbonates rather than carbonates, and that these compounds have distinct and improved properties. It has also been found that modifications of the preparation method create different porous physical structures with improved properties.

The compounds of the present invention and in particular, the lanthanum compounds and more particularly the lanthanum oxycarbonates of the present invention exhibit phosphate binding or removal of at least 40% of the initial concentration of phosphate after ten minutes. Desirably, the lanthanum compounds exhibit phosphate binding or removal of at least 60% of the initial concentration of phosphate after ten minutes. In other words, the lanthanum compounds and in particular, the lanthanum compounds and more particularly the lanthanum oxycarbonates of the present invention exhibit a phosphate binding capacity of at least 45 mg of phosphate per gram of lanthanum compound. Suitably, the lanthanum compounds exhibit a phosphate binding capacity of at least 50 mg $PO_4$/g of lanthanum compound, more suitably, a phosphate binding capacity of at least 75 mg $PO_4$/g of lanthanum compound. Desirably, the lanthanum compounds exhibit a phosphate binding capacity of at least 100 mg $PO_4$/g of lanthanum compound, more desirably, a phosphate binding capacity of at least 10 mg $PO_4$/g of lanthanum compound.

In accordance with the present disclosure, rare earth metal compounds, and in particular, rare earth metal oxychlorides and oxycarbonates are provided. The oxycarbonates may be hydrated or anhydrous. These compounds may be produced as particles having a porous structure. The rare earth metal compound particles may conveniently be produced within a controllable range of surface areas with resultant variable and controllable adsorption rates of ions.

The porous particles or porous structures of the present disclosure are made of nano-sized to micron-sized crystals with controllable surface areas. In an embodiment not covered by the claimed invention, the rare earth oxychloride is desirably lanthanum oxychloride (LaOCl). In another embodiment not covered by the claimed invention, therare earth oxycarbonate hydrate is desirably lanthanum oxycarbonate hydrate ($La_2O(CO_3)_2$•$xH_2O$ where x is from and including 2 to and including 4). This compound will further be referred to in this text as $La_2O(CO_3)_2$•$xH_2O$. The anhydrous rare earth oxycarbonate is desirably lanthanum oxycarbonate $La_2O_2CO_3$ or $La_2CO_5$ of which several crystalline forms exist. The lower temperature form will be identified as $La_2O_2CO_3$ and the form obtained at higher temperature or after a longer calcination time will be identified as $La_2CO_5$.

One skilled in the art, however, will understand that lanthanum oxycarbonate may be present as a mixture of the hydrate and the anhydrous form. In addition, the anhydrous lanthanum oxycarbonate may be present as a mixture of $La_2O_2CO_3$ and $La_2CO_5$ and may be present in more than a single crystalline form.

One method of making the rare earth metal compound particles, not covered by the claimed invention, includes making a solution of rare earth metal chloride, subjecting the solution to a substantially total evaporation process using a spray dryer or other suitable equipment to form an intermediate product, and calcining the obtained intermediate product at a temperature between about 500° and about 1200°C. The product of the calcination step may be washed, filtered, and dried to make a suitable finished product. Optionally, the intermediate product may be milled in a horizontal or vertical pressure media mill to a desired surface area and then further spray dried or dried by other means to produce a powder that may be further washed and filtered.

An alternative method of making the rare earth metal compounds not covered by the claimed invention, particularly rare earth metal anhydrous oxycarbonate particles includes making a solution of rare earth metal acetate, subjecting the solution to a substantially total evaporation process using a spray dryer or other suitable equipment to make an intermediate product, and calcining the obtained intermediate product at a temperature between about 400°C and about 700°C. The product of the calcination step may be washed, filtered, and dried to make a suitable finished product. Optionally, the intermediate product may be milled in a horizontal or vertical pressure media mill to a desired surface area, spray dried or dried by other means to produce a powder that may be washed, filtered, and dried.

Yet another method of making the rare earth metal compounds not covered by the claimed invention includes making rare earth metal oxycarbonate hydrate particles. The rare earth metal oxycarbonate hydrate particles can be made by successively making a solution of rare earth chloride, subjecting the solution to a slow, steady feed of a sodium carbonate solution at a temperature between about 30° and about 90°C while mixing, then filtering and washing the precipitate to form a filter cake, then drying the filter cake at a temperature of about 100° to 120°C to produce the desired rare earth oxycarbonate hydrate species. Optionally, the filter cake may be sequentially dried, slurried, and milled in a horizontal or vertical pressure media mill to a desired surface area, spray dried or dried by other means to produce a powder that may be washed, filtered, and dried.

Alternatively, the process for making rare earth metal oxycarbonate hydrate particles may be modified to produce anhydrous particles. This modification includes subjecting the dried filter cake to a thermal treatment at a specified temperature between about 400°C to about 700°C and for a specified time between 1h and 48h. Optionally, the product of the thermal treatment may be slurried and milled in a horizontal or vertical pressure media mill to a desired surface area, spray dried or dried by other means to produce a powder that may be washed, filtered, and dried.

In accordance with the present invention, compounds of the present invention may be used to treat patients with hyperphosphatemia. The compounds may be made into a form that may be delivered to a mammal and that may be used to remove phosphate from the gut or decrease phosphate absorption into the blood stream. For example, the compounds may be formulated to provide an orally ingestible form such as a liquid solution or suspension, a tablet, capsule, gelcap, or other suitable and known oral form. Accordingly, the present invention contemplates a method for treating hyperphosphatemia that comprises providing an effective amount of a compound of the present invention. Compounds made under different conditions will correspond to different oxycarbonates or oxychlorides, will have different surface areas, and will show differences in reaction rates with phosphate and different solubilization of lanthanum or another rare-earth metal into the gut. The present invention allows one to modify these properties according to the requirements of the treatment.

In another aspect of the present disclosure not covered by the claimed invention, compounds made according to this disclosure as a porous structure of sufficient mechanical strength may be placed in a device fluidically connected to a dialysis machine through which the blood flows, to directly remove phosphate by reaction of the rare-earth compound with phosphate in the bloodstream. The present embodiment not covered by the claimed invention therefore contemplates a device having an inlet and an outlet with one or more compounds of the present invention disposed between the inlet and the outlet. The present embodiment not covered by the claimed invention also contemplates a method of reducing the amount of phosphate in blood that comprises contacting the blood with one or more compounds of the present invention for a time sufficient to reduce the amount of phosphate in the blood.

In yet another aspect of the present disclosure not covered by the claimed invention, the compounds of the present disclosure may be used as a substrate for a filter having an inlet and outlet such that the compounds of the present disclosure are disposed between the inlet and the outlet. A fluid containing a metal, metal ion, phosphate or other ion may be passed from the inlet to contact the compounds of the present disclosure and through the outlet. Accordingly, in one aspect of the present disclosure not covered by the claimed invention a method of reducing the content of a metal in a fluid, for example water, comprises flowing the fluid through a filter that contains one or more compounds of the present disclosure to reduce the amount of metal present in the water.

## BRIEF DESCRIPTION OF THE DRAWINGS

FIG. 1 is a general flow sheet of a process that produces LaOCl (lanthanum oxychloride).

FIG. 2 is a flow sheet of a process that produces a coated titanium dioxide structure.

FIG. 3 is a flow sheet of a process that produces lanthanum oxycarbonate.

FIG. 4 is a graph showing the percentage of phosphate removed from a solution as a function of time by $LaO(CO_3)_2 \cdot x\ H_2O$, (where x is from and including 2 to and including 4), made according to the process of the present disclosure, but not covered by the claimed invention, as compared to the percentage of phosphate removed by commercial grade La carbonate $La_2(CO_3)_3 \cdot 4H_2O$ in the same conditions.

FIG. 5 is a graph showing the amount of phosphate removed from a solution as a function of time per g of a lanthanum compound used as a drug to treat hyperphosphatemia. The drug in one case is $La_2O(CO_3)_2 \cdot x\ H_2O$ (where x is from and including 2 to and including 4), made according to the disclosed process (not covered by the claimed invention). In the comparative case the drug is commercial grade La carbonate $La_2(CO_3)_3 \cdot 4H_2O$.

FIG. 6 is a graph showing the amount of phosphate removed from a solution as a function of time per g of a lanthanum compound used as a drug to treat hyperphosphatemia. The drug in one case is $La_2O_2CO_3$ made according to the process of the present invention. In the comparative case the drug is commercial grade La carbonate $La_2(CO_3)_3 \cdot 4H_2O$.

FIG. 7 is a graph showing the percentage of phosphate removed as a function of time by $La_2O_2CO_3$ made according to the process of the present invention, as compared to the percentage of phosphate removed by commercial grade La carbonate $La_2(CO_3)_3 \cdot 4H_2O$.

FIG. 8 is a graph showing a relationship between the specific surface area of the oxycarbonates made following the process of the present invention and the amount of phosphate bound or removed from solution 10 min after the addition of the oxycarbonate.

FIG. 9 is a graph showing a linear relationship between the specific surface area of the oxycarbonates of this invention and the first order rate constant calculated from the initial rate of reaction of phosphate.

FIG. 10 is a flow sheet of a process that produces lanthanum oxycarbonate hydrate $La_2(CO_3)_2 \cdot xH_2O$.

FIG. 11 is a flow sheet of a process according to the present invention that produces anhydrous lanthanum oxycarbonate $La_2O_2CO_3$ or $La_2CO_5$.

FIG. 12 is a scanning electron micrograph of lanthanum oxychloride, made following the process of the present disclosure not covered by the claimed invention.

FIG. 13 is an X-Ray diffraction scan of lanthanum oxychloride LaOCl made according to the process of the present disclosure not covered by the claimed invention and compared with a standard library card of lanthanum oxychloride.

FIG. 14 is a graph showing the percentage of phosphate removed from a solution as a function of time by LaOCl made according to the process of the present disclosure not covered by the claimed invention, as compared to the amount of phosphate removed by commercial grades of La carbonate $La_2(CO_3)_3 \cdot H_2O$ and $La_2(CO_3)_3 \cdot 4H_2O$ in the same conditions.

FIG. 15 shows a scanning electron micrograph of $La_2O(CO_3)_2 \cdot x\,H_2O$, where x is from and including 2 to and including 4.

FIG. 16 is an X-Ray diffraction scan of $La_2O(CO_3)_2 \cdot x\,H_2O$ produced according to the present disclosure not covered by the claimed invention and includes a comparison with a "library standard" of $La_2O(CO_3)_2 \cdot xH_2O$ where x is from and including 2 to and including 4.

FIG. 17 is a graph showing the rate of removal of phosphorous from a solution by $La_2O(CO_3)_2 \cdot xH_2O$ compared to the rate obtained with commercially available $La_2(CO_3)_3 \cdot H_2O$ and $La_2(CO_3)_3 \cdot 4H_2O$ in the same conditions.

FIG. 18 is a scanning electron micrograph of anhydrous lanthanum oxycarbonate $La_2O_2CO_3$.

FIG. 19 is an X-Ray diffraction scan of anhydrous $La_2O_2CO_3$ produced according to the present invention and includes a comparison with a "library standard" of $La_2O_2CO_3$.

FIG. 20 is a graph showing the rate of phosphorous removal obtained with $La_2O_2CO_3$ made following the process of the present invention and compared to the rate obtained for commercially available $La_2(CO_3)_3 \cdot H_2O$ and $La_2(CO_3)_3 \cdot 4H_2O$.

FIG. 21 is a scanning electron micrograph of $La_2CO_5$ made according to the process of the present invention.

FIG. 22 is an X-Ray diffraction scan of anhydrous $La_2CO_5$ produced according to the present invention and includes a comparison with a "library standard" of $La_2CO_5$.

FIG. 23 is a graph showing the rate of phosphorous removal obtained with $La_2CO_5$ made following the process of the present invention and compared to the rate obtained for commercially available $La_2(CO_3)_3 \cdot H_2O$ and $La_2(CO_3)_3 \cdot 4H_2O$.

FIG. 24 is a scanning electron micrograph of Ti02 support material made according to the process of the present disclosure not covered by the claimed invention.

FIG. 25 is a scanning electron micrograph of a Ti02 structure coated with LaOCl, made according to the process of the present disclosure not covered by the claimed invention, calcined at 800 °C.

FIG. 26 is a scanning electron micrograph of a Ti02 structure coated with LaOCl, made according to the process of the present disclosure not covered by the claimed invention, calcined at 600 °C.

FIG. 27 is a scanning electron micrograph of a Ti02 structure coated with LaOCl, made according to the process of the present disclosure not covered by the claimed invention, calcined at 900°C.

FIG. 28. shows X-Ray scans for $TiO_2$ coated with LaOCl and calcined at different temperatures following the process of the present disclosure not covered by the claimed invention, and compared to the X-Ray scan for pure LaOCl.

FIG. 29 shows the concentration of lanthanum in blood plasma as a function of time, for dogs treated with lanthanum oxycarbonates made according to the process of the present invention.

FIG. 30 shows the concentration of phosphorous in urine as a function of time in rats treated with lanthanum oxycarbonates made according to the process of the present invention, and compared to phosphorus concentration measured in untreated rats.

FIG. 31 shows a device, not covered by the claimed invention, having an inlet, an outlet, and one or more compounds of the present disclosure disposed between the inlet and the outlet.

## DESCRIPTION OF THE INVENTION

Referring now to the drawings, the process of the present invention will be described.

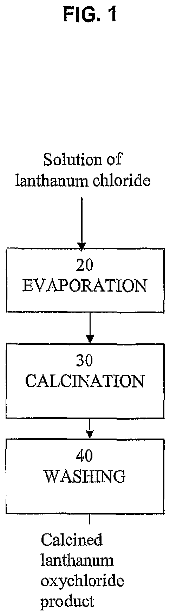

Turning now to FIG. 1, a process for making a rare earth oxychloride compound, and, in particular a lanthanum oxychloride compound according to one embodiment of the present disclosure not covered by the claimed invention is shown. First, a solution of lanthanum chloride is provided. The source of lanthanum chloride may be any suitable source and is not limited to any particular source. One source of lanthanum chloride solution is to dissolve commercial lanthanum chloride crystals in water or in an HCl solution. Another source is to dissolve lanthanum oxide in a hydrochloric acid solution.

The lanthanum chloride solution is evaporated to form an intermediate product. The evaporation 20 is conducted under conditions to achieve substantially total evaporation. Desirably, the evaporation is conducted at a temperature higher than the boiling point of the feed solution (lanthanum chloride) but lower than the temperature where significant crystal growth occurs. The resulting intermediate product may be an amorphous solid formed as a thin film or may have a spherical shape or a shape as part of a sphere.

The terms "substantially total evaporation" or "substantially complete evaporation" as used in the specification and claims refer to evaporation such that the resulting solid intermediate contains less than 15% free water, desirably less than 10% free water, and more desirably less than 1% free water. The term "free water" is understood and means water that is not chemically bound and can be removed by heating at a temperature below 150°C. After substantially total evaporation or substantially complete evaporation, the intermediate product will have no visible moisture present.

The evaporation step may be conducted in a spray dryer. In this case, the intermediate product will consist of a structure of spheres or parts of spheres. The spray dryer generally operates at a discharge temperature between about 120°C and about 500°C.

The intermediate product may then be calcined in any suitable calcination apparatus 30 by raising the temperature to a temperature between about 500°C to about 1200°C for a period of time from about 2 to about 24 h and then cooling to room temperature. The cooled product may be washed 40 by immersing it in water or dilute acid, to remove any water-soluble phase that may still be present after the calcination step 30.

The temperature and the length of time of the calcination process may be varied to adjust the particle size and the reactivity of the product. The particles resulting from calcination generally have a size between 1 and 1000 $\mu$m. The calcined particles consist of individual crystals, bound together in a structure with good physical strength and a porous structure. The individual crystals forming the particles generally have a size between 20 nm and 10 $\mu$m.

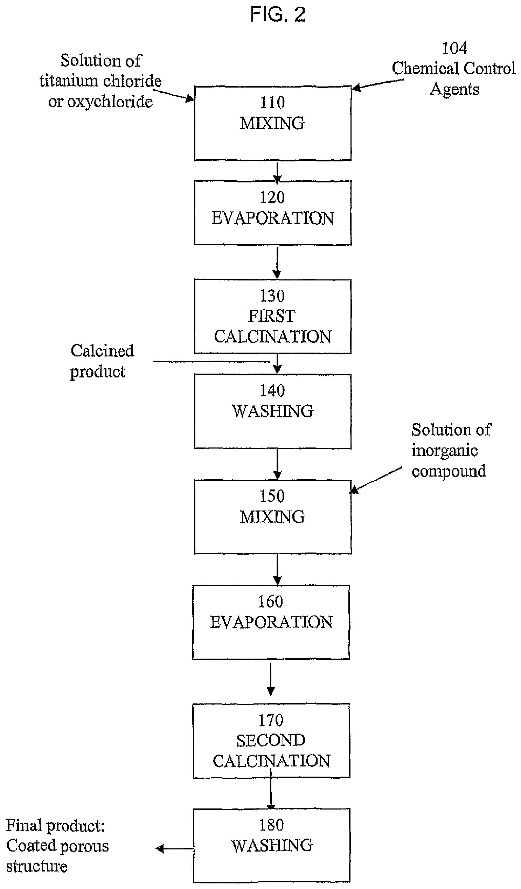

In accordance with another embodiment of the present disclosure, not covered by the claimed invention, as shown in FIG. 2, a feed solution of titanium chloride or titanium oxychloride is provided by any suitable source. One source is to dissolve anhydrous titanium chloride in water or in a hydrochloric acid solution. Chemical control agents or additives 104 may be introduced to this feed solution to influence the crystal form and the particle size of the final product. One chemical additive is sodium phosphate $Na_3PO_4$. The feed solution of titanium chloride or titanium oxychloride is mixed with the optional chemical control agent 104 in a suitable mixing step 110. The mixing may be conducted using any suitable known mixer.

The feed solution is evaporated to form an intermediate product, which in this instance is titanium dioxide ($TiO_2$). The evaporation 120 is conducted at a temperature higher than the boiling point of the feed solution but lower than the temperature where significant crystal growth occurs and to achieve substantially total evaporation. The resulting intermediate product may desirably be an amorphous solid formed as a thin film and may have a spherical shape or a shape as part of a sphere.

The intermediate product may then be calcined in any suitable calcination apparatus 130 by raising the temperature to a temperature between about 400°C to about 1200°C for a period of time from about 2 to about 24 h and then cooling to room temperature (25°C). The cooled product is then washed 140 by immersing it in water or dilute acid, to remove traces of any water-soluble phase that may still be present after the calcination step.

The method of manufacture of the intermediate product according to the present disclosure, not covered by the claimed invention, can be adjusted and chosen to make a structure with the required particle size and porosity. For example, the evaporation step 120 and the calcination step 130 can be adjusted for this purpose. The particle size and porosity can be adjusted to make the structure of the intermediate product suitable to be used as an inert filter in the bloodstream.

The washed $TiO_2$ product is then suspended or slurried in a solution of an inorganic compound. A desirable inorganic compound is a rare-earth or lanthanum compound, and in particular lanthanum chloride. This suspension of $TiO_2$ in the inorganic compound solution is again subjected to total evaporation 160 under conditions in the same range as defined in step 120 and to achieve substantially total evaporation. In this regard, the evaporation steps 120 and 160 may be conducted in a spray drier. The inorganic compound will precipitate as a salt, an oxide, or an oxy-salt. If the inorganic compound is lanthanum chloride, the precipitated product will be lanthanum oxychloride. If the original compound is lanthanum acetate, the precipitated product will be lanthanum oxide.

The product of step 160 is further calcined 170 at a temperature between 500° and 1100 °C for a period of 2 to 24 h. The temperature and the time of the calcination process influence the properties and the particle size of the product. After the second calcination step 170, the product may be washed 180.

The resulting product can be described as crystals of lanthanum oxychloride or lanthanum oxide formed on a $TiO_2$ substrate. The resulting product may be in the form of hollow thin-film spheres or parts of spheres. The spheres will have a size of about 1 $\mu$m to 1000 $\mu$m and will consist of a structure of individual bound particles. The individual particles have a size between 20 nm and 10 $\mu$m.

When the final product consists of crystals of lanthanum oxychloride on a $TiO_2$ substrate, these crystals may be hydrated. It has been found that this product will effectively react with phosphate and bind it as an insoluble compound. It is believed that, if this final product is released in the human stomach and gastrointestinal tract, the product will bind the phosphate that is present and decrease the transfer of phosphate from the stomach and gastrointestinal tract to the blood stream. Therefore, the product of this disclosure not covered by the claimed invention may be used to limit the phosphorous content in the bloodstream of patients on kidney dialysis.

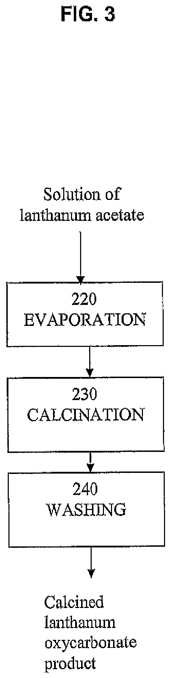

According to another embodiment of the present disclosure not covered by the claimed invention, a process for making anhydrous lanthanum oxycarbonate is shown in FIG. 3. In this process, a solution of lanthanum acetate is made by any method. One method to make the lanthanum acetate solution is to dissolve commercial lanthanum acetate crystals in water or in an HCl solution.

The lanthanum acetate solution is evaporated to form an intermediate product. The evaporation 220 is conducted at a temperature higher than the boiling point of the lanthanum acetate solution but lower than the temperature where significant crystal growth occurs and under conditions to achieve substantially total evaporation. The resulting intermediate product may desirably be an amorphous solid formed as a thin film and may have a spherical shape or a shape as part of a sphere.

The intermediate product may then be calcined in any suitable calcination apparatus 230 by raising the temperature to a temperature between about 400°C to about 800°C for a period of time from about 2 to about 24 h and then cooled to room temperature. The cooled product may be washed 240 by immersing it in water or dilute acid, to remove any water-soluble phase that may still be present after the calcination step. The temperature and the length of time of the calcination process may be varied to adjust the particle size and the reactivity of the product.

The particles resulting from the calcination generally have a size between 1 and 1000 $\mu$m. The calcined particles consist of individual crystals, bound together in a structure with good physical strength and a porous structure. The individual crystals generally have a size between 20 nm and 10 $\mu$m.

The products made by methods shown in FIGs. 1, 2, and 3 comprise ceramic particles with a porous structure. Individual particles are in the micron size range. The particles are composed of crystallites in the nano-size range, fused together to create a structure with good strength and porosity.

The particles made according to the process of the present disclosure not covered by the claimed invention, have the following common properties:

a. They have low solubility in aqueous solutions, especially serum and gastro-intestinal fluid, compared to non-ceramic compounds.

b. Their hollow shape gives them a low bulk density compared to solid particles. Lower density particles are less likely to cause retention in the gastro-intestinal tract.

c. They have good phosphate binding kinetics. The observed kinetics are generally better than the commercial carbonate hydrates $La_2(CO_3)_3 \cdot H_2O$ and $La_2(CO_3)_3 \cdot 4H_2O$. In the case of lanthanum oxychloride, the relationship between the amount of phosphate bound or absorbed and time tends to be closer to linear than for commercial hydrated lanthanum carbonates. The initial reaction rate is lower but does not significantly decrease with time over an extended period. This behavior is defined as linear or substantially linear binding kinetics. This is probably an indication of more selective phosphate binding in the presence of other anions.

d. Properties a, b, and c, above are expected to lead to less gastro-intestinal tract complications than existing products.

e. Because of their particular structure and low solubility, the products have the potential to be used in a filtration device placed directly in the bloodstream.

Different lanthanum oxycarbonates have been prepared by different methods. It has been found that, depending on the method of preparation, lanthanum oxycarbonate compounds with widely different reaction rates are obtained.

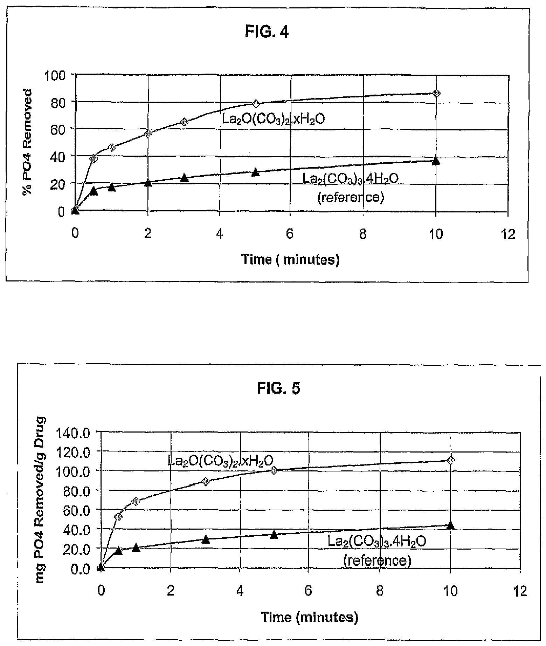

A desirable lanthanum oxycarbonate not covered by the claimed invention is $La_2O(CO_3)_2 \cdot xH_2O$, where $2 \leq x \leq 4$. This lanthanum oxycarbonate is preferred because it exhibits a relatively high rate of removal of phosphate. To determine the reactivity of the lanthanum oxycarbonate compound with respect to phosphate, the following procedure was used. A stock solution containing 13.75 g/l of anhydrous $Na_2HPO_4$ and 8.5 g/l of HCl is prepared. The stock solution is adjusted to pH 3 by the addition of concentrated HCl. 100 ml of the stock solution is placed in a beaker with a stirring bar. A sample of lanthanum oxycarbonate powder is added to the solution. The amount of lanthanum oxycarbonate powder is such that the amount of La in suspension is 3 times the stoichiometric amount needed to react completely with the phosphate. Samples of the suspension are taken at intervals, through a filter that separated all solids from the liquid. The liquid sample is analyzed for phosphorous. FIG. 4 shows that after 10 min, $La_2O(CO_3)_2 \cdot xH_2O$ has removed 86% of the phosphate in solution, whereas a commercial hydrated La carbonate $La_2(CO_3)_3 \cdot 4H_2O$ removes only 38% of the phosphate in the same experimental conditions after the same time.

FIG. 5 shows that the $La_2O(CO_3)_2 \cdot xH_2O$ depicted in Fig. 4 has a capacity of phosphate removal of 110 mg $PO_4$ removed/g of La compound after 10 min in the conditions described above, compared to 45 mg $PO_4$/g for the commercial La carbonate taken as reference.

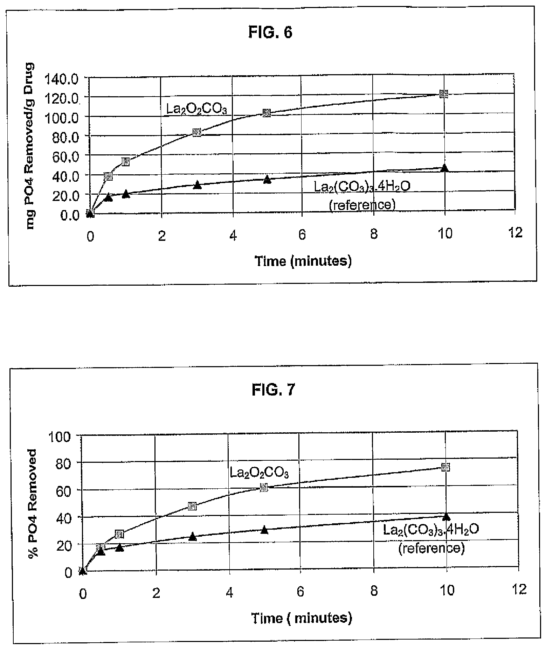

Another preferred lanthanum carbonate is the anhydrous La oxycarbonate $La_2O_2CO_3$. This compound is preferred because of its particularly high binding capacity for phosphate, expressed as mg $PO_4$ removed/ g of compound. FIG. 6 shows that $La_2O_2CO_3$ binds 120 mg $PO_4$/g of La compound after 10 min, whereas $La_2(CO_3)_3 \cdot 4H_2O$ used as reference only binds 45 mg $PO_4$/g La compound.

Fig. 7 shows the rate of reaction with phosphate of the oxycarbonate $La_2O_2CO_3$. After 10 min of reaction, 73% of the phosphate had been removed, compared to 38% for commercial lanthanum carbonate used as reference.

Samples of different oxycarbonates have been made by different methods as shown in Table 1 below.

Table 1

| Sample | Compound | Example number corresponding to manufacturing method | BET surface area $m^2/g$ | Fraction of $PO_4$ remaining after 10 min | Initial 1 st order rate constant $k_1$ ($min^{-1}$) |
|---|---|---|---|---|---|
| 1 | $La_2O(CO_3)_2 \cdot xH_2O$ | 11 | 41.3 | 0.130 | 0.949 |
| 2 | $La_2O(CO_3)_2 \cdot xH_2O$ | 11 | 35.9 | 0.153 | 0.929 |
| 3 | $La_2O(CO_3)_2 \cdot xH_2O$ | 11 | 38.8 | 0.171 | 0.837 |
| 4 | $La_2CO_5$ (4 h milling) | 7 | 25.6 | 0.275 | 0.545 |
| 5 | $La_2O_2CO_3$ | 5 | 18 | 0.278 | 0.483 |
| 6 | $La_2CO_5$ (2 h milling) | 7 | 18.8 | 0.308 | 0.391 |
| 7 | $La_2O_2CO_3$ | 7 | 16.5 | 0.327 | 0.36 |
| 8 | $La_2CO_5$ (no milling) | 5 | 11.9 | 0.483 | 0.434 |
| 9 | $La_2(CO_3)_3 \cdot 4H_2O$ | commercial sample | 4.3 | 0.623 | 0.196 |
| 10 | $La_2(CO_3)_3 \cdot 1H_2O$ | commercial | 2.9 | 0.790 | 0.094 |

For each sample, the surface area measured by the BET method and the fraction of phosphate remaining after 10 min of reaction have been tabulated. The table also shows the rate constant $k_1$ corresponding to the initial rate of reaction of phosphate, assuming the reaction is first order in phosphate concentration. The rate constant $k_1$ is defined by the following equation:

$$d[PO_4]/dt = -k_1 [PO_4]$$

where $[PO_4]$ is the phosphate concentration in solution (mol/liter), t is time (min) and $k_1$ is the first order rate constant ($min^{-1}$). The table gives the rate constant for the initial reaction rate, i.e. the rate constant calculated from the experimental points for the first minute of the reaction.

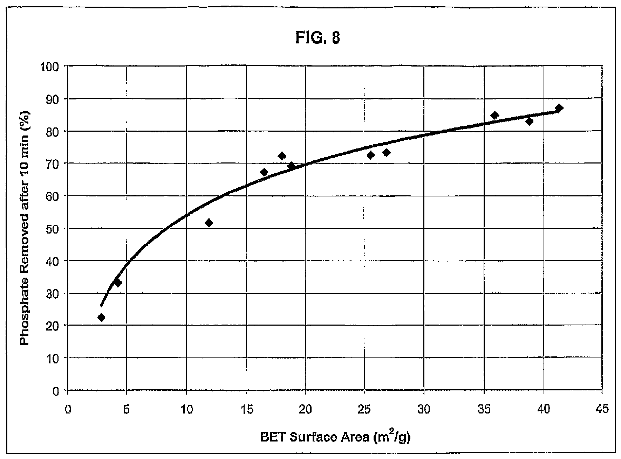

FIG. 8 shows that there is a good correlation between the specific surface area and the amount of phosphate reacted after 10 min. It appears that in this series of tests, the most important factor influencing the rate of reaction is the surface area, independently of the composition of the oxycarbonate or the method of manufacture. A high surface area can be achieved by adjusting the manufacturing method or by milling a manufactured product.

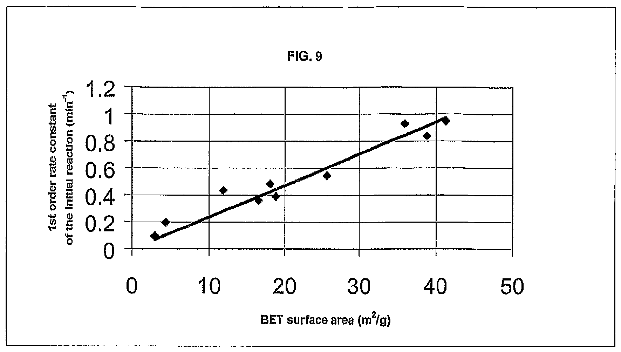

FIG. 9 shows that a good correlation is obtained for the same compounds by plotting the first order rate constant as given in Table I and the BET specific surface area. The correlation can be represented by a straight line going through the origin. In other words, within experimental error, the initial rate of reaction appears to be proportional to the phosphate concentration and also to the available surface area.

Without being bound by any theory, it is proposed that the observed dependence on surface area and phosphate concentration may be explained by a nucleophilic attack of the phosphate ion on the La atom in the oxycarbonate, with resultant formation of lanthanum phosphate $LaPO_4$. For example, if the oxycarbonate is $La_2O_2CO_3$, the reaction will be:

$$\tfrac{1}{2} La_2O_2CO_3 + PO_4^{3-} + 2H_2O \rightarrow LaPO_4 + \tfrac{1}{2} H_2CO_3 + 3OH^-$$

If the rate is limited by the diffusion of the $PO_4^{3-}$ ion to the surface of the oxycarbonate and the available area of oxycarbonate, the observed relationship expressed in FIG. 9 can be explained. This mechanism does not require La to be present as a dissolved species. The present reasoning also provides an explanation for the decrease of the reaction rate after the first minutes: the formation of lanthanum phosphate on the surface of the oxycarbonate decreases the area available for reaction.

In general, data obtained at increasing pH show a decrease of the reaction rate. This may be explained by the decrease in concentration of the hydronium ion ($H_3O^+$), which may catalyze the reaction by facilitating the formation of the carbonic acid molecule from the oxycarbonate.

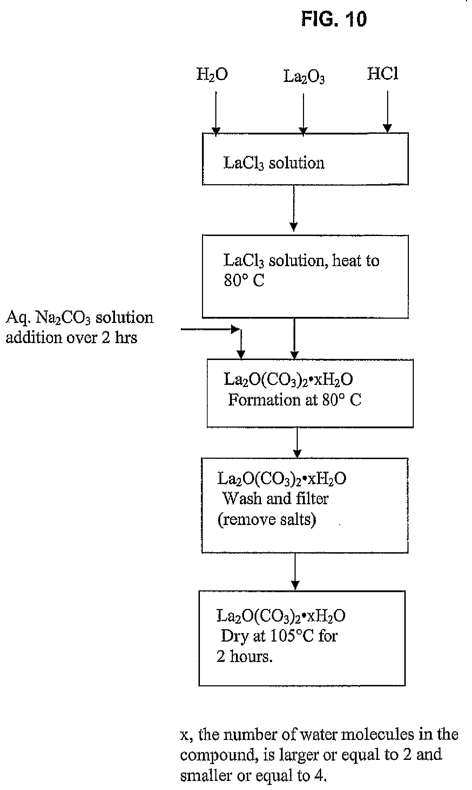

Turning now to FIG. 10, another process for making lanthanum oxycarbonate, not covered by the claimed invention, and in particular, lanthanum oxycarbonate tetra hydrate, is shown. First, an aqueous solution of lanthanum chloride is made by any method. One method to make the solution is to dissolve commercial lanthanum chloride crystals in water or in an HCl solution. Another method to make the lanthanum chloride solution is to dissolve lanthanum oxide in a hydrochloric acid solution.

The $LaCl_3$ solution is placed in a well-stirred tank reactor. The $LaCl_3$ solution is then heated to 80°C. A previously prepared analytical grade sodium carbonate is steadily added over a period of 2 hours with vigorous mixing. The mass of sodium carbonate required is calculated at 6 moles of sodium carbonate per 2 moles of $LaCl_3$. When the required mass of sodium carbonate solution is added, the resultant slurry or suspension is allowed to cure for 2 hours at 80°C. The suspension is then filtered and washed with demineralized water to produce a clear filtrate. The filter cake is placed in a convection oven at 105° C for 2 hours or until a stable weight is observed. The initial pH of the $LaCl_3$ solution is 2, while the final pH of the suspension after cure is 5.5. A white powder is produced. The resultant powder is a lanthanum oxycarbonate four hydrate ($La_2O(CO_3)_2 \cdot xH_2O$). The number of water molecules in this compound is approximate and may vary between 2 and 4 (and including 2 and 4).

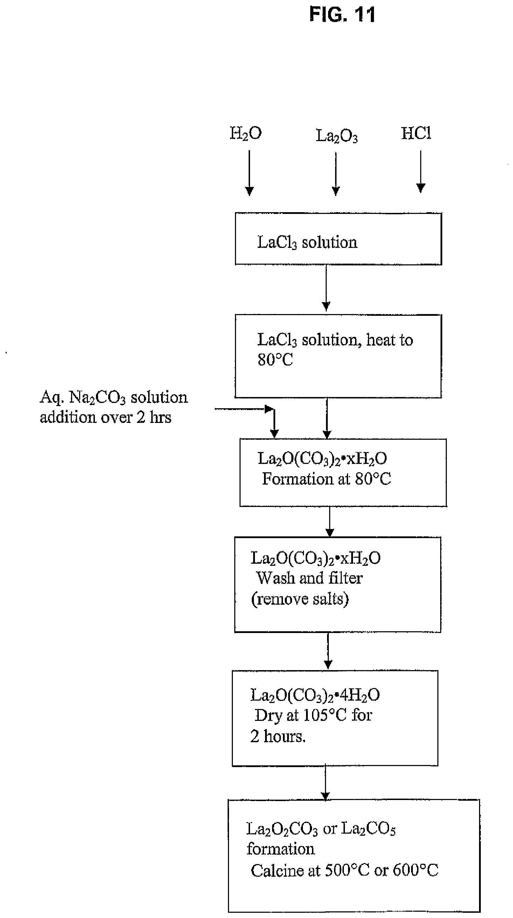

Turning now to FIG. 11a process for making anhydrous lanthanum oxycarbonate according to an embodiment of the invention is shown. First, an aqueous solution of lanthanum chloride is made by any method. One method to make the solution is to dissolve commercial lanthanum chloride crystals in water or in an HCl solution. Another method to make the lanthanum chloride solution is to dissolve lanthanum oxide in a hydrochloric acid solution.

The $LaCl_3$ solution is placed in a well-stirred tank reactor. The $LaCl_3$ solution is then heated to 80°C. A previously prepared analytical grade sodium carbonate is steadily added over 2 hours with vigorous mixing. The mass of sodium carbonate required is calculated at 6 moles of sodium carbonate per 2 moles of $LaCl_3$. When the required mass of sodium carbonate solution is added the resultant slurry or suspension is allowed to cure for 2 hours at 80°C. The suspension is then washed and filtered removing NaCl (a byproduct of the reaction) to produce a clear filtrate. The filter cake is placed in a convection oven at 105°C for 2 hours or until a stable weight is observed. The initial pH of the $LaCl_3$ solution is 2.2, while the final pH of the suspension after cure is 5.5. A white lanthanum oxycarbonate hydrate powder is produced. Next the lanthanum oxycarbonate hydrate is placed in an alumina tray, which is placed in a high temperature muffle furnace. The white powder is heated to 500°C and held at that temperature for 3 hours. Anhydrous $La_2C_2O_3$ is formed.

Alternatively, the anhydrous lanthanum oxycarbonate formed as indicated in the previous paragraph may be heated at 500 °C for 15 to 24 h instead of 3h or at 600 °C instead of 500 °C. The resulting product has the same chemical formula, but shows a different pattern in an X-Ray diffraction scan and exhibits a higher physical strength and a lower surface area. The product corresponding to a higher temperature or a longer calcination time is defined here as $La_2CO_5$.

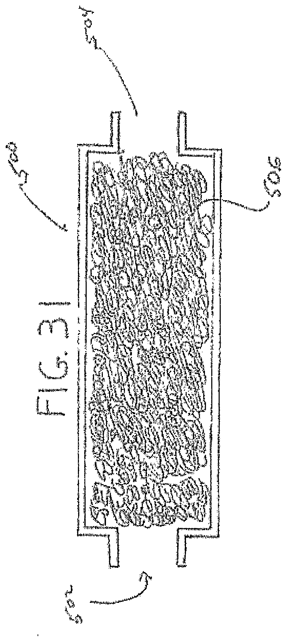

Turning now to FIG. 31, a device 500, not covered by the claimed invention, having an inlet 502 and an outlet 504 is shown. The device 500 may be in the form of a filter or other suitable container. Disposed between the inlet 502 and the outlet 504 is a substrate 506 in the form of a plurality of one or more compounds of the present invention. The device may be fluidically connected to a dialysis machine through which the blood flows, to directly remove phosphate by reaction of the rare-earth compound with phosphate in the bloodstream. In this connection, the present disclosure also contemplates a method of reducing the amount of phosphate in blood that comprises contacting the blood with one or more compounds of the present disclosure for a time sufficient to reduce the amount of phosphate in the blood.

In yet another aspect of the present invention, the device 500 may be provided in a fluid stream so that a fluid containing a metal, metal ion, phosphate or other ion may be passed from the inlet 502 through the substrate 506 to contact the compounds of the present disclosure and out the outlet 504. Accordingly, in one aspect of the present disclosure not covered by the claimed invention a method of reducing the content of a metal in a fluid, for example water, comprises flowing the fluid through a device 500 that contains one or more compounds of the present disclosure to reduce the amount of metal present in the water.

The following examples are meant to illustrate but not limit the present disclosure.

## EXAMPLE 1

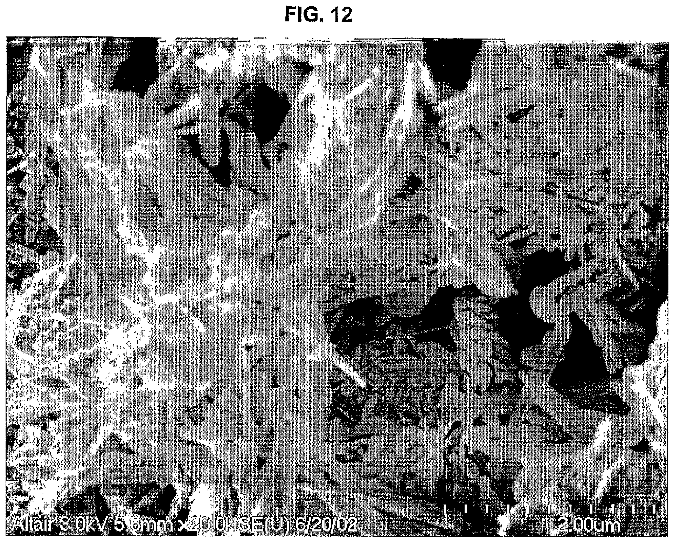

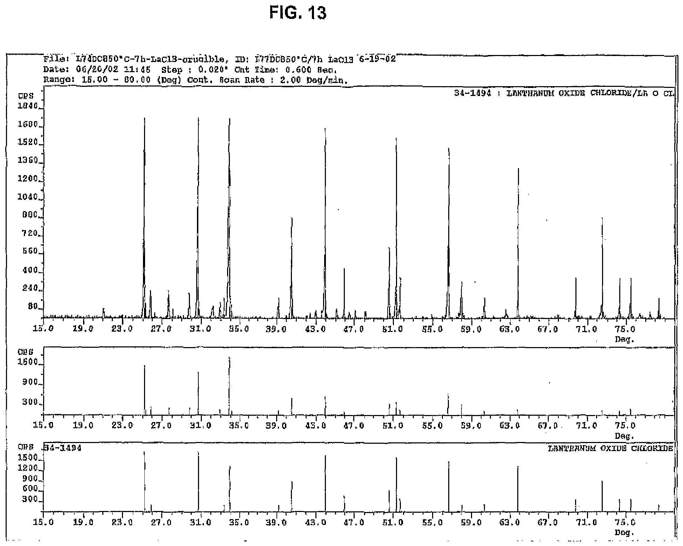

An aqueous solution containing 100 g/l of La as lanthanum chloride is injected in a spray dryer with an outlet temperature of 250 °C. The intermediate product corresponding to the spray-drying step is recovered in a bag filter. This intermediate product is calcined at 900°C for 4 hours. FIG. 12 shows a scanning electron micrograph of the product, enlarged 25,000 times. The micrograph shows a porous structure formed of needle-like particles. The X-Ray diffraction pattern of the product (FIG. 13) shows that it consists of lanthanum oxychloride LaOCl.

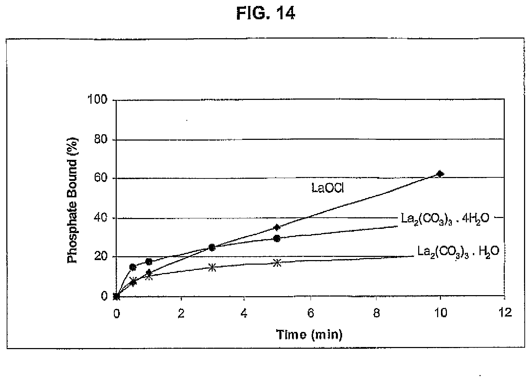

To determine the reactivity of the lanthanum compound with respect to phosphate, the following test was conducted. A stock solution containing 13.75 g/l of anhydrous $Na_2HPO_4$ and 8.5 g/l of HCl was prepared. The stock solution was adjusted to pH 3 by the addition of concentrated HCl. An amount of 100 ml of the stock solution was placed in a beaker with a stirring bar. The lanthanum oxychloride from above was added to the solution to form a suspension. The amount of lanthanum oxychloride was such that the amount of La in suspension was 3 times the stoichiometric amount needed to react completely with the phosphate. Samples of the suspension were taken at time intervals, through a filter that separated all solids from the liquid. The liquid sample was analyzed for phosphorous. FIG. 14 shows the rate of phosphate removed from solution.

**EXAMPLE 2 (Comparative example)**

To determine the reactivity of a commercial lanthanum with respect to phosphate, the relevant portion of Example 1 was repeated under the same conditions, except that commercial lanthanum carbonate $La_2(CO_3)_3 \cdot H_2O$ and $La_2(CO_3)_3 \cdot 4H_2O$ was used instead of the lanthanum oxychloride of the present disclosure not covered by the claimed invention. Additional curves on FIG. 14 show the rate of removal of phosphate corresponding to commercial lanthanum carbonate $La_2(CO_3)_3 \cdot H_2O$ and $La_2(CO_3)_3 \cdot 4H_2O$. FIG. 14 shows that the rate of removal of phosphate with the commercial lanthanum carbonate is faster at the beginning but slower after about 3 minutes.

**EXAMPLE 3**

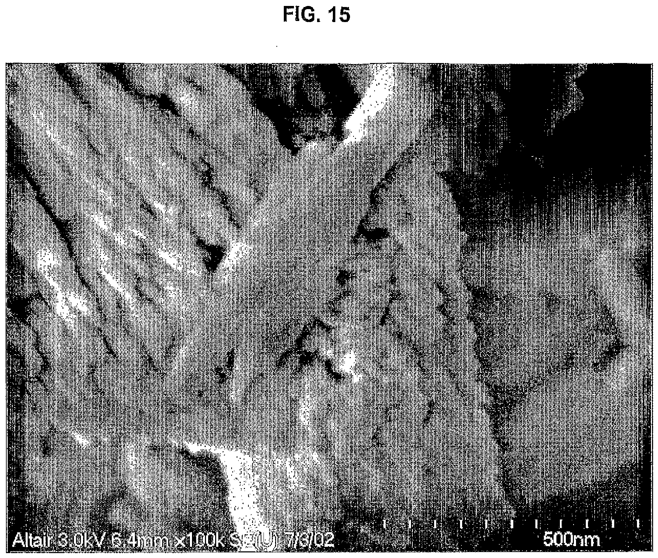

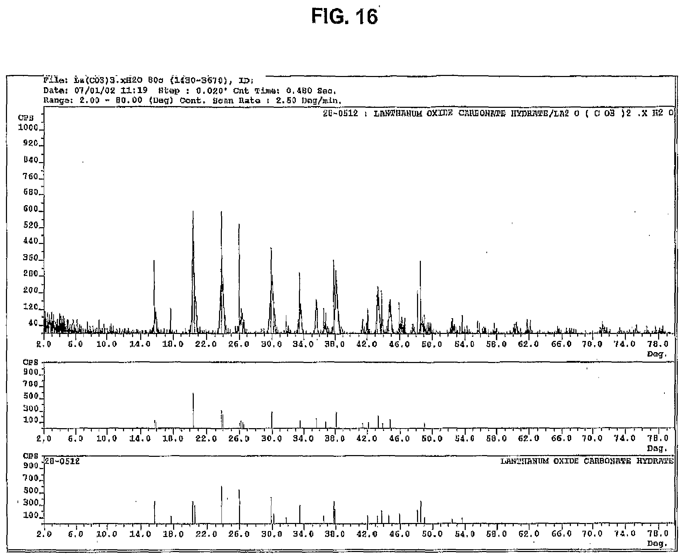

An aqueous HCl solution having a volume of 334.75 ml and containing $LaCl_3$ (lanthanum chloride) at a concentration of 29.2 wt % as $La_2O_3$ was added to a four liter beaker and heated to 80°C with stirring. The initial pH of the $LaCl_3$ solution was 2.2. Two hundred and sixty five ml of an aqueous solution containing 63.59 g of sodium carbonate $(Na_2CO_3)$ was metered into the heated beaker using a small pump at a steady flow rate for 2 hours. Using a Buchner filtering apparatus fitted with filter paper, the filtrate was separated from the white powder product. The filter cake was mixed four times with 2 liters of distilled water and filtered to wash away the NaCl formed during the reaction. The washed filter cake was placed into a convection oven set at 105° C for 2 hours, or until a stable weight was observed. FIG. 15 shows a scanning electron micrograph of the product, enlarged 120,000 times. The micrograph shows the needle-like structure of the compound. The X-Ray diffraction pattern of the product (FIG. 16) shows that it consists of hydrated lanthanum oxycarbonate hydrate $(La_2O(CO_3)_2 \cdot xH_2O)$, with $2 \leq x \leq 4$.

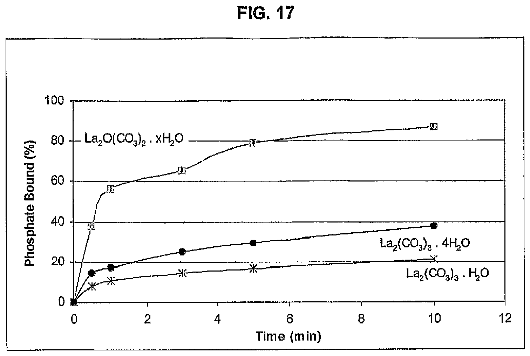

To determine the reactivity of the lanthanum compound with respect to phosphate, the following test was conducted. A stock solution containing 13.75 g/l of anhydrous $Na_2HPO_4$ and 8.5 g/l of HCl was prepared. The stock solution was adjusted to pH 3 by the addition of concentrated HCl. An amount of 100 ml of the stock solution was placed in a beaker with a stirring bar. Lanthanum oxycarbonate hydrate powder made as described above was added to the solution. The amount of lanthanum oxycarbonate hydrate powder was such that the amount of La in suspension was 3 times the stoichiometric amount needed to react completely with the phosphate. Samples of the suspension were taken at time intervals through a filter that separated all solids from the liquid. The liquid sample was analyzed for phosphorous. FIG. 17 shows the rate of phosphate removed from solution.

**EXAMPLE 4 (Comparative example)**

To determine the reactivity of a commercial lanthanum with respect to phosphate, the second part of Example 3 was repeated under the same conditions, except that commercial lanthanum carbonate $La_2(CO_3)_3 \cdot H_2O$ and $La_2(CO_3)_3 \cdot 4H_2O$ was used instead of the lanthanum oxychloride of the present disclosure not covered by the claimed invention. FIG. 17 shows the rate of phosphate removed using the commercial lanthanum carbonate $La_2(CO_3)_3 \cdot H_2O$ and $La_2(CO_3)_3 \cdot 4H_2O$. FIG. 17 shows that the rate of removal of phosphate with the lanthanum oxycarbonate is faster than with the commercial lanthanum carbonate hydrate $(La_2(CO_3)_3 \cdot H_2O$ and $La_2(CO_3)_3 \cdot 4H_2O)$.

**EXAMPLE 5**

An aqueous HCl solution having a volume of 334.75 ml and containing $LaCl_3$ (lanthanum chloride) at a concentration of 29.2 wt % as $La_2O_3$ was added to a 4 liter beaker and heated to 80°C with stirring. The initial pH of the $LaCl_3$ solution was 2.2. Two hundred and sixty five ml of an aqueous solution containing 63.59 g of sodium carbonate $(Na_2CO_3)$ was metered into the heated beaker using a small pump at a steady flow rate for 2 hours. Using a Buchner filtering apparatus fitted with filter paper the filtrate was separated from the white powder product. The filter cake was mixed four times with 2 liters of distilled water and filtered to wash away the NaCl formed during the reaction. The washed filter cake was placed into a convection oven set at 105°C for 2 hours until a stable weight was observed. Finally, the lanthanum oxycarbonate was placed in an alumina tray in a muffle furnace. The furnace temperature was ramped to 500°C and held at that temperature for 3 hours. The resultant product was determined to be anhydrous lanthanum oxycarbonate $La_2O_2CO_3$.

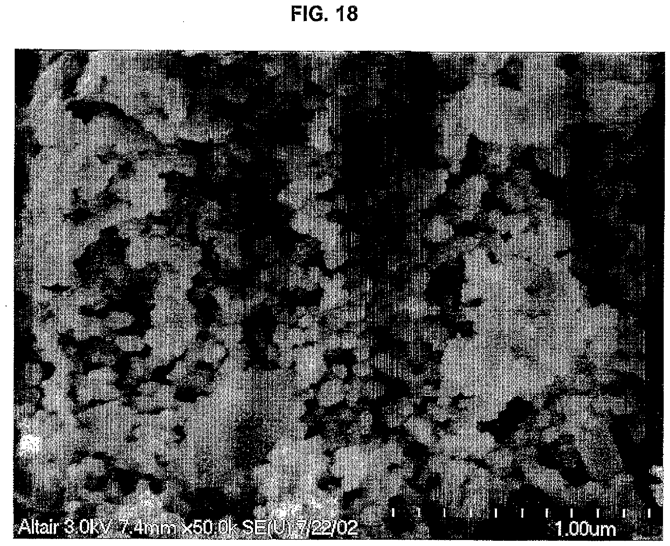

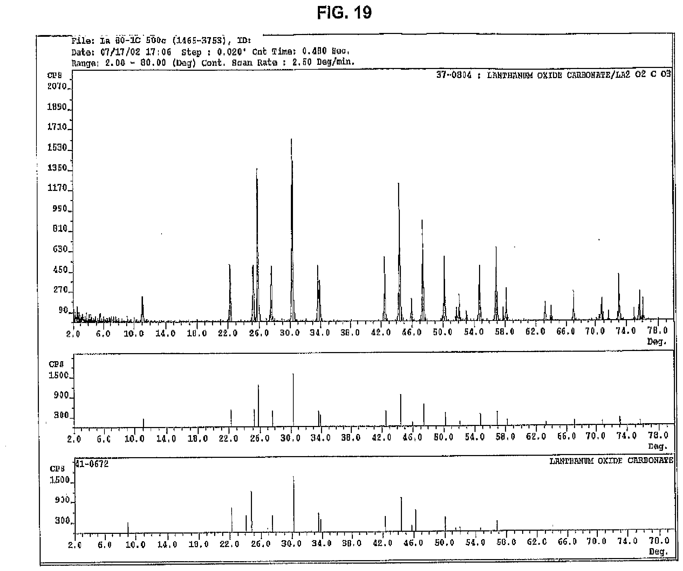

The process was repeated three times. In one case, the surface area of the white powder was determined to be 26.95 $m^2$/gm. In the other two instances, the surface area and reaction rate is shown in Table 1. FIG. 18 is a scanning electron micrograph of the structure, enlarged 60,000 times. The micrograph shows that the structure in this compound is made of equidimensional or approximately round particles of about 100 nm in size. FIG. 19 is an X-ray diffraction pattern showing that the product made here is an anhydrous lanthanum oxycarbonate written as $La_2O_2CO_3$.

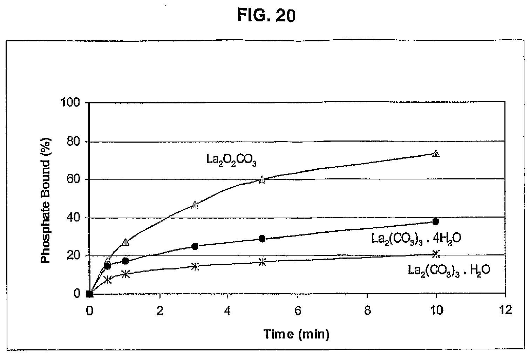

To determine the reactivity of this lanthanum compound with respect to phosphate, the following test was conducted. A stock solution containing 13.75 g/l of anhydrous $Na_2HPO_4$ and 8.5 g/l of HCl was prepared. The stock solution was adjusted to pH 3 by the addition of concentrated HCl. An amount of 100 ml of the stock solution was placed in a beaker with a stirring bar. Anhydrous lanthanum oxycarbonate made as described above, was added to the solution. The amount of anhydrous lanthanum oxycarbonate was such that the amount of La in suspension was 3 times the stoichiometric amount needed to react completely with the phosphate. Samples of the suspension were taken at intervals, through a filter that separated all solids from the liquid. The liquid sample was analyzed for phosphorous. FIG. 20 shows the rate of phosphate removed.

**EXAMPLE 6 (Comparative example)**

To determine the reactivity of a commercial lanthanum with respect to phosphate, the second part of Example 5 was repeated under the same conditions, except that commercial lanthanum carbonate $La_2(CO_3)_3 \cdot H_2O$ and $La_2(CO_3)_3 \cdot 4H_2O$ was used instead of the $La_2O_2CO_3$ of the present invention. FIG. 20 shows the rate of removal of phosphate using the commercial lanthanum carbonate $La_2(CO_3)_3 \cdot H_2O$ and $La_2(CO_3)_3 \cdot 4H_2O$. FIG. 20 shows that the rate of removal of phosphate with the anhydrous lanthanum oxycarbonate produced according to the process of the present invention is faster than the rate observed with commercial lanthanum carbonate hydrate $La_2(CO_3)_3 \cdot H_2O$ and $La_2(CO_3)_3 \cdot 4H_2O$.

**EXAMPLE 7**

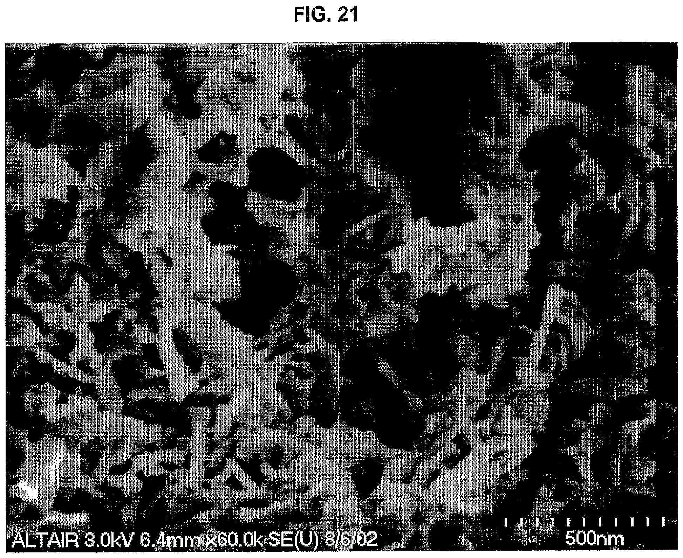

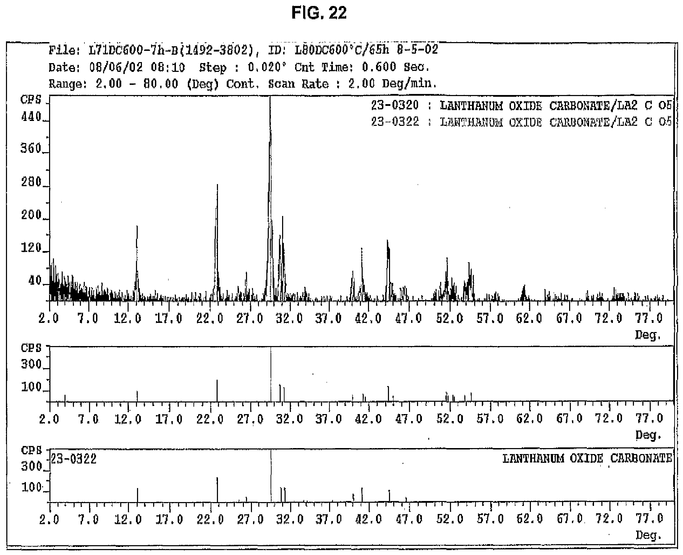

A solution containing 100 g/l of La as lanthanum acetate is injected in a spray-drier with an outlet temperature of 250°C. The intermediate product corresponding to the spray-drying step is recovered in a bag filter. This intermediate product is calcined at 600°C for 4 hours. FIG. 21 shows a scanning electron micrograph of the product, enlarged 80,000 times. FIG. 22 shows the X-Ray diffraction pattern of the product and it shows that it consists of anhydrous lanthanum oxycarbonate. The X-Ray pattern is different from the pattern corresponding to Example 5, even though the chemical composition of the compound is the same. The formula for this compound is written as $(La_2CO_5)$. Comparing FIGS. 21 and 18 shows that the compound of the present example shows a structure of leaves and needles as opposed to the round particles formed in Example 5. The particles may be used in a device to directly remove phosphate from an aqueous or nonaqueous medium, e.g., the gut or the bloodstream.

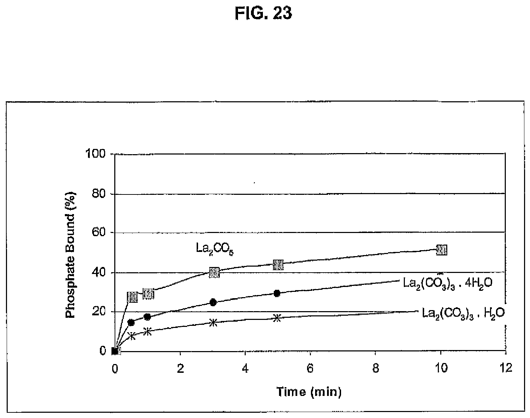

To determine the reactivity of the lanthanum compound with respect to phosphate, the following test was conducted. A stock solution containing 13.75 g/l of anhydrous $Na_2HPO_4$ and 8.5 g/l of HCl was prepared. The stock solution was adjusted to pH 3 by the addition of concentrated HCl. An amount of 100 ml of the stock solution was placed in a beaker with a stirring bar. $La_2CO_5$ powder, made as described above, was added to the solution. The amount of lanthanum oxycarbonate was such that the amount of La in suspension was 3 times the stoichiometric amount needed to react completely with the phosphate. Samples of the suspension were taken at intervals through a filter that separated all solids from the liquid. The liquid sample was analyzed for phosphorous. FIG. 23 shows the rate of phosphate removed from solution.

**EXAMPLE 8 (Comparative example)**

To determine the reactivity of a commercial lanthanum with respect to phosphate commercial lanthanum carbonate $La_2(CO_3)_3 \cdot H_2O$ and $La_2(CO_3)_3 \cdot 4H_2O$ was used instead of the lanthanum oxycarbonate made according to the present invention as described above. FIG. 23 shows the rate of phosphate removal for the commercial lanthanum carbonate $La_2(CO_3)_3 \cdot H_2O$ and $La_2(CO_3)_3 \cdot 4H_2O$. FIG. 23 also shows that the rate of phosphate removal with the lanthanum oxycarbonate is faster than the rate of phosphate removal with commercial lanthanum carbonate hydrate $La_2(CO_3)_3 \cdot H_2O$ and $La_2(CO_3)_3 \cdot 4H_2O$.

**EXAMPLE 9**

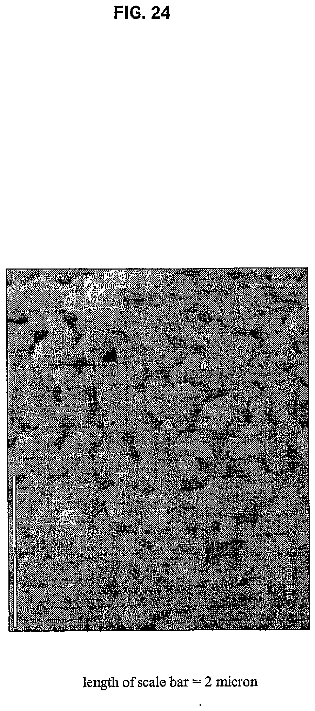

To a solution of titanium chloride or oxychloride containing 120 g/l Ti and 450 g/l Cl is added the equivalent of 2.2 g/l of sodium phosphate $Na_3PO_4$. The solution is injected in a spray dryer with an outlet temperature of 250 °C. The spray dryer product is calcined at 1050 °C for 4 h. The product is subjected to two washing steps in 2 molar HCl and to two washing steps in water. FIG. 24 is a scanning electron micrograph of the $TiO_2$ material obtained. It shows a porous structure with individual particles of about 250 nm connected in a structure. This structure shows good mechanical strength. This material can be used as an inert filtering material in a fluid stream such as blood.

**EXAMPLE 10**

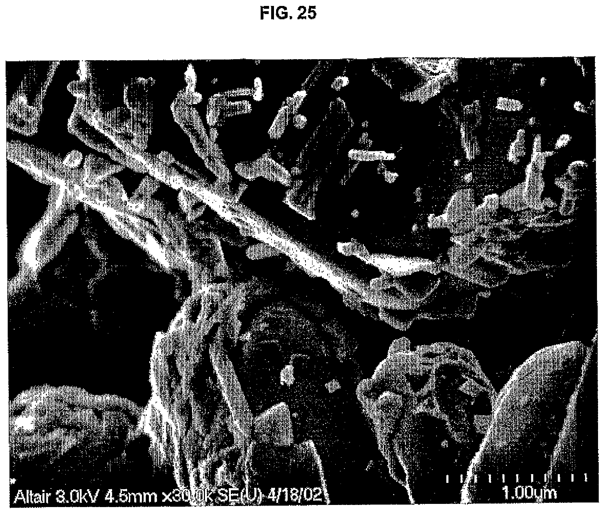

The product of Example 9 is re-slurried into a solution of lanthanum chloride containing 100 g/l La. The slurry contains approximately 30% $TiO_2$ by weight. The slurry is spray dried in a spray dryer with an outlet temperature of 250° C. The product of the spray drier is further calcined at 800° C for 5 h. It consists of a porous $TiO_2$ structure with a coating of nano-sized lanthanum oxychloride. FIG. 25 is a scanning electron micrograph of this coated product. The electron micrograph shows that the $TiO_2$ particles are several microns in size. The LaOCl is present as a crystallized deposit with elongated crystals, often about 1 $\mu$m long and 0.1 $\mu$m across, firmly attached to the $TiO_2$ catalyst support surface as a film of nano-size thickness. The LaOCl growth is controlled by the $TiO_2$ catalyst support structure. Orientation of rutile crystals works as a template for LaOCl crystal growth. The particle size of the deposit can be varied from the nanometer to the micron range by varying the temperature of the second calcination step.

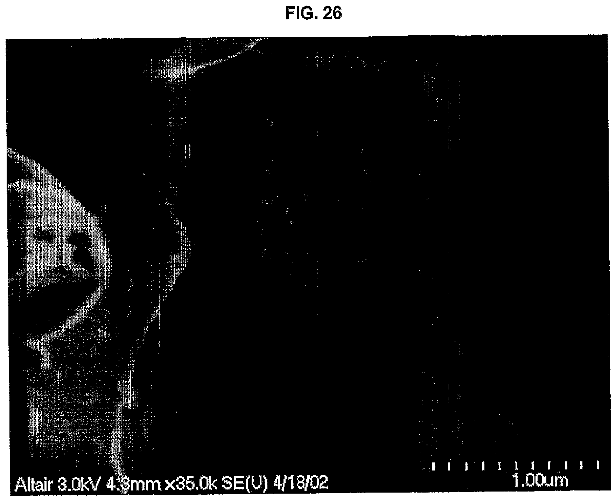

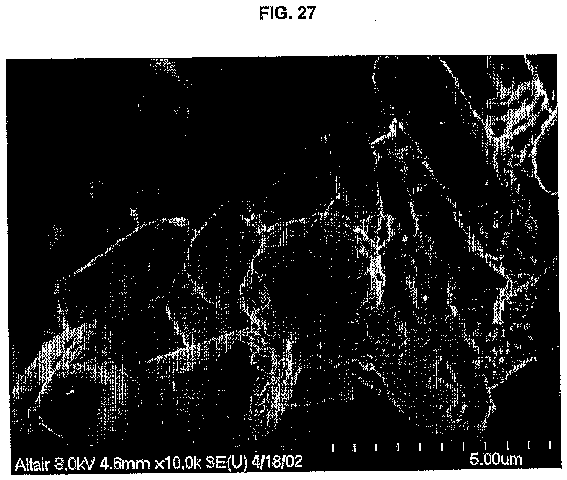

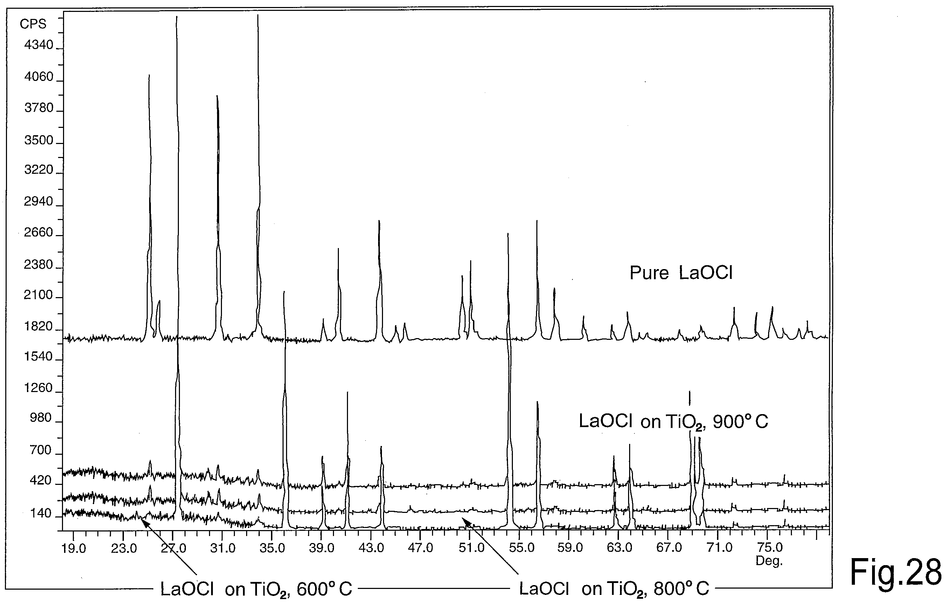

FIG. 26 is a scanning electron micrograph corresponding to calcination at 600° C instead of 800° C. It shows LaOCl particles that are smaller and less well attached to the $TiO_2$ substrate. FIG. 27 is a scanning electron micrograph corresponding to calcination at 900°C instead of 800 °C. The product is similar to the product made at 800° C, but the LaOCl deposit is present as somewhat larger crystals and more compact layer coating the Ti02 support crystals. FIG. 28 shows the X-Ray diffraction patterns corresponding to calcinations at 600°, 800° and 900° C. The figure also shows the pattern corresponding to pure LaOCl. The peaks that do not appear in the pure LaOCl pattern correspond to rutile $TiO_2$. As the temperature increases, the peaks tend to become higher and narrower, showing that the crystal size of the LaOCl as well as $TiO_2$ increases with the temperature.

**EXAMPLE 11**

An aqueous HCl solution having a volume of 334.75 ml and containing $LaCl_3$ (lanthanum chloride) at a concentration of 29.2 wt % as $La_2O_3$ was added to a 4 liter beaker and heated to 80°C with stirring. The initial pH of the $LaCl_3$ solution was 2.2. Two hundred and sixty five ml of an aqueous solution containing 63.59 g of sodium carbonate ($Na_2CO_3$) was metered into the heated beaker using a small pump at a steady flow rate for 2 hours. Using a Buchner filtering apparatus fitted with filter paper the filtrate was separated from the white powder product. The filter cake was mixed four times, each with 2 liters of distilled water and filtered to wash away the NaCl formed during the reaction. The washed filter cake was placed into a convection oven set at 105 °C for 2 hours or until a stable weight was observed. The X-Ray diffraction pattern of the product shows that it consists of hydrated lanthanum oxycarbonate $La_2O(CO_3)_2 \cdot xH_2O$, where $2 \leq x \leq 4$. The surface area of the product was determined by the BET method. The test was repeated 3 times and slightly different surface areas and different reaction rates were obtained as shown in Table 1.

**EXAMPLE 12**

Six adult beagle dogs were dosed orally with capsules of lanthanum oxycarbonate $La_2O(CO_3)_2 \cdot xH_2O$ (compound A) or $La_2O_2CO_3$ (compound B) in a crossover design using a dose of 2250 mg elemental lanthanum twice daily (6 hours apart). The doses were administered 30 minutes after provision of food to the animals. At least 14 days washout was allowed between the crossover arms. Plasma was obtained pre-dose and 1.5, 3, 6, 7.5, 9, 12, 24, 36, 48, 60, and 72 hours after dosing and analyzed for lanthanum using ICP-MS. Urine was collected by catheterization before and approximately 24 hours after dosing and creatinine and phosphorus concentrations measured.

The tests led to reduction of urine phosphate excretion, a marker of phosphorous binding. Values of phosphate excretion in urine are shown in Table 2 below.

Table 2

| La Oxycarbonate compound | Median phosphorus/creatinine ratio (% reduction compared to pre-dose value) | 10th and 90th percentiles |
|---|---|---|
| A | 48.4% | 22.6-84.4% |
| B | 37.0% | -4.1-63.1% |

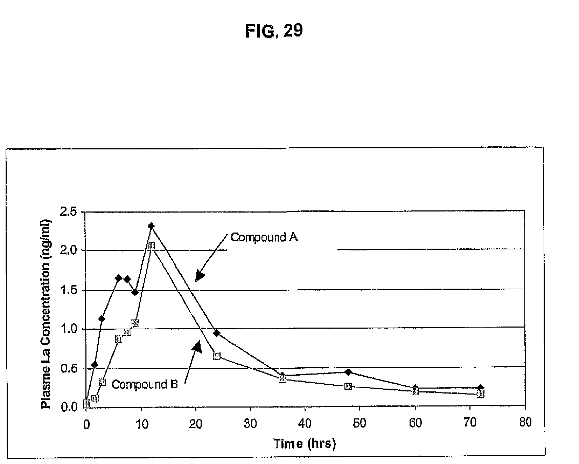

Plasma lanthanum exposure: Overall plasma lanthanum exposure in the dogs is summarized in Table 3 below. The plasma concentration curves are shown in FIG. 29.

Table 3

| La oxycarbonate compound tested | Mean (sd) Area Under the Curve$_{0-72h}$ (ng.h/mL); (standard deviation) | Maximum concentration Cmax (ng/mL); (standard deviation) |
|---|---|---|
| A | 54.6 (28.0) | 2.77(2.1) |
| B | 42.7 (34.8) | 2.45 (2.2) |

## EXAMPLE 13 - First *in vivo* study in rats

Groups of six adult Sprague-Dawley rats underwent 5/6th nephrectomy in two stages over a period of 2 weeks and were then allowed to recover for a further two weeks prior to being randomized for treatment. The groups received vehicle (0.5% w/v carboxymethyl cellulose), or lanthanum oxycarbonate A or B suspended in vehicle, once daily for 14 days by oral lavage (10ml/kg/day). The dose delivered 314 mg elemental lanthanum/kg/day. Dosing was carried out immediately before the dark (feeding) cycle on each day. Urine samples (24 hours) were collected prior to surgery, prior to the commencement of treatment, and twice weekly during the treatment period. Volume and phosphorus concentration were measured.

Feeding- During the acclimatization and surgery period, the animals were given Teklad phosphate sufficient diet (0.5% Ca, 0.3%P; Teklad No. TD85343), ad libitum. At the beginning of the treatment period, animals were pair fed based upon the average food consumption of the vehicle-treated animals the previous week.

5/6 Nephrectomy - After one week of acclimatization, all animals were subjected to 5/6 nephrectomy surgery. The surgery was performed in two stages. First, the two lower branches of the left renal artery were ligated. One week later, a right nephrectomy was performed. Prior to each surgery, animals were anesthetized with an intra-peritoneal injection of ketamine/xylazine mixture (Ketaject a 100mg/ml and Xylaject at 20mg/ml) administered at 10 ml/kg. After each surgery, 0.25 mg/kg Buprenorphine was administered for relief of post-surgical pain. After surgery, animals were allowed to stabilize for 2 weeks to beginning treatment.

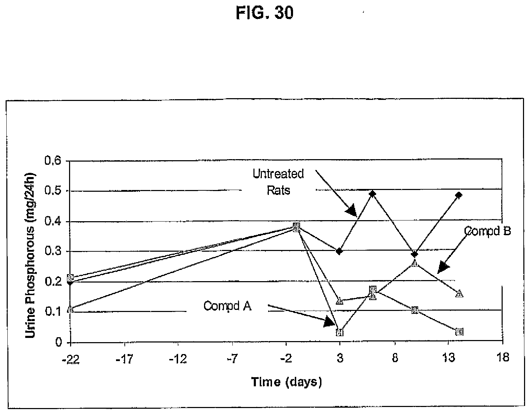

The results showing urine phosphorus excretion are given in FIG. 30. The results show a decrease in phosphorus excretion, a marker of dietary phosphorus binding, after administration of the lanthanum oxycarbonate (at time > 0), compared to untreated rats.

## EXAMPLE 14: Second *in vivo* study in rats

Six young adult male Sprague-Dawley rats were randomly assigned to each group. Test items were lanthanum oxycarbonates $La_2O_2CO_3$ and $La_2CO_5$ (compound B and compound C), each tested at 0.3 and 0.6% of diet. There was an additional negative control group receiving Sigmacell cellulose in place of the test item.

The test items were mixed thoroughly into Teklad 7012CM diet. All groups received equivalent amounts of dietary nutrients.

Table 4 outlines the dietary composition of each group:

**Table 4**

| Group ID | Treatment | Test Item | Sigmacell cellulose | Teklad Diet |
|---|---|---|---|---|
| I | Negative control | 0.0% | 1.2% | 98.8% |
| II | Compound B - Mid level | 0.3% | 0.9% | 98.8% |
| III | Compound B - High level | 0.6% | 0.6% | 98.8% |
| IV | Compound C - Mid level | 0.3% | 0.9% | 98.8% |
| V | Compound C - High level | 0.6% | 0.6% | 98.8% |

Rats were maintained in the animal facility for at least five days prior to use, housed individually in stainless steel hanging cages. On the first day of testing, they were placed individually in metabolic cages along with their test diet. Every 24 hours, their output of urine and feces was measured and collected and their general health visually assessed. The study continued for 4 days. Food consumption for each day of the study was recorded. Starting and ending animal weights were recorded.

Plasma samples were collected via retro-orbital bleeding from the control (I) and high-dose oxycarbonate groups, III and V. The rats were then euthanized with $CO_2$ in accordance with the IACUC study protocol.

Urine samples were assayed for phosphorus, calcium, and creatinine concentration in a Hitachi 912 analyzer using Roche reagents. Urinary excretion of phosphorus per day was calculated for each rat from daily urine volume and phosphorus concentration. No significant changes were seen in animal weight, urine volume or creatinine excretion between groups. Food consumption was good for all groups.

Even though lanthanum dosage was relatively low compared to the amount of phosphate in the diet, phosphate excretion for 0.3 or 0.6% La added to the diet decreased as shown in Table 5 below. Table 5 shows average levels of urinary phosphate over days 2, 3, and 4 of the test. Urine phosphorus excretion is a marker of dietary phosphorous binding.

Table 5

|  | Urinary phosphate excretion (mg/day) |
|---|---|
| Control | 4.3 |
| Compound B =$La_2O_2CO_3$ | 2.3 |
| Compound C = $La_2CO_5$ | 1.9 |

**EXAMPLE 15:**

Tests were run to determine the binding efficiency of eight different compounds for twenty-four different elements. The compounds tested are given in Table 6.

Table 6

| Test ID | Compound | Preparation Technique |
|---|---|---|
| 1 | $La_2O_3$ | Calcined the commercial (Prochem) $La_2(CO_3)_3 \cdot H_2O$ at 850°C for 16hrs. |
| 2 | $La_2CO_5$ | Prepared by spray drying lanthanum acetate solution and calcining at 600°C for 7hrs (method corresponding to FIG. 3) |
| 3 | LaOCl | Prepared by spray drying lanthanum chloride solution and calcining at 700°C for 10hrs (method corresponding to FIG, 1) |
| 4 | $La_2(CO_3)_3 \cdot 4H_2O$ | Purchased from Prochem (comparative example) |
| 5 | Ti carbonate | Made by the method of FIG. 11, where the $LaCl_3$ solution is replaced by a $TiOCl_2$ solution. |
| 6 | $TiO_2$ | Made by the method corresponding to FIG. 2, with addition of sodium chloride. |
| 7 | $La_2O(CO_3)_2 \cdot xH_2O$ | Precipitation by adding sodium carbonate solution to lanthanum chloride solution at 80°C (Method corresponding to FIG. 10) |
| 8 | $La_2O_2CO_3$ | Precipitation by adding sodium carbonate solution to lanthanum chloride solution at 80°C followed by calcination at 500°C for 3hrs. (Method of FIG. 11) |

The main objective of the tests was to investigate the efficiency at which the compounds bind arsenic and selenium, in view of their use in removing those elements from drinking water. Twenty-one different anions were also included to explore further possibilities. The tests were performed as follows:

The compounds given in Table 6 were added to water and a spike and were vigorously shaken at room temperature for 18hrs. The samples were filtered and the filtrate analyzed for a suite of elements including Sb, As, Be, Cd, Ca, Cr, Co, Cu, Fe, Pb, Mg, Mn, Mo, Ni, Se, Tl, Ti, V, Zn, Al, Ba, B, Ag, and P.

The spike solution was made as follows:

1. in a 500ml volumetric cylinder add 400ml of de-Ionized water.
2. Add standard solutions of the elements given above to make solutions containing approximately 1 mg/l of each element.
3. Dilute to 500mls with de-ionized water.

The tests were conducted as follows:

1. Weigh 0.50g of each compound into its own 50ml centrifuge tube.
2. Add 30.0ml of the spike solution to each.
3. Cap tightly and shake vigorously for 18hrs.
4. Filter solution from each centrifuge tube through $0.2\mu$m syringe filter. Obtain ~6ml of filtrate.
5. Dilute filtrates 5:10 with 2% $HNO_3$. Final Matrix is 1% $HNO_3$.
6. Submit for analysis.

The results of the tests are given in Table 7.

Table 7

| | \% of the Analyte Removed | | | | | | | | | | | | | | | | | | | | | | | |
|---|---|---|---|---|---|---|---|---|---|---|---|---|---|---|---|---|---|---|---|---|---|---|---|---|
| | Sb | As | Be | Cd | Ca | Cr | Co | Cu | Fe | Pb | Mg | Mn | Mo | Ni | Se | Tl | Ti | V | Zn | Al | Ba | B | Ag | P |
| $La_2O_3$ | 89 | 85 | 97 | 95 | 21 | **100** | 69 | 89 | 92 | 92 | 0 | 94 | 89 | 28 | 72 | 8 | 90 | 95 | 95 | 85 | 23 | 0 | 47 | 96 |
| $La_2CO_5$ | **96** | 93 | **100** | 83 | 0 | **100** | 52 | 97 | 100 | 99 | 0 | 99 | 98 | 17 | 79 | 8 | 100 | 99 | 100 | 93 | 0 | 0 | 73 | 99 |
| $LaOCl$ | 86 | 76 | 89 | 46 | 0 | **100** | 28 | 88 | 100 | 99 | 0 | 28 | 94 | 0 | 71 | 13 | 100 | 99 | 24 | 92 | 7 | 0 | 96 | 96 |
| $La_2(CO_3)_3 \cdot 4H_2O$ | 84 | 25 | 41 | 37 | 28 | **94** | 20 | 0 | 56 | 90 | 0 | 20 | 98 | 1 | 78 | 5 | 100 | 99 | 16 | 11 | 23 | 0 | 48 | 71 |
| $Ti(CO_3)_2$ | **96** | 93 | **100** | 100 | 99 | **99** | **99** | 98 | 100 | 98 | 79 | **1 00** | 91 | 98 | 97 | 96 | 24 | 100 | 100 | 92 | 100 | 0 | 99 | 98 |
| $TiO_2$ | **96** | **93** | 8 | 4 | 0 | 6 | 0 | 11 | 49 | 97 | 0 | 1 | 97 | 0 | 97 | 62 | 0 | 86 | 0 | 0 | 0 | 30 | **99** | 66 |
| $La_2O(CO_3)_2 \cdot xH_2O$ | 87 | 29 | 53 | 37 | 28 | **100** | 20 | 10 | 58 | 98 | 0 | 25 | 99 | 0 | 79 | 8 | **100** | 99 | 16 | 60 | 26 | 0 | 44 | 74 |
| $La_2O_2CO_3$ | **97** | 92 | **100** | 85 | 21 | **100** | 59 | **98** | 100 | 99 | 0 | **99** | 99 | 34 | 81 | 12 | **100** | 99 | **100** | 92 | 23 | 0 | 87 | 99 |

The most efficient compounds for removing both arsenic and selenium appear to be the titanium-based compounds 5 and 6. The lanthanum oxycarbonates made according to the process of the present invention remove at least 90% of the arsenic. Their efficiency at removing Se is in the range 70 to 80%. Commercial lanthanum carbonate (4 in Table 6) is less effective.

The tests show that the lanthanum and titanium compounds made following the process of the present invention are also effective at removing Sb, Cr, Pb, Mo from solution. They also confirm the efficient removal of phosphorus discussed in the previous examples.

## Claims

1.  A compound selected from the group consisting of crystalline anhydrous $La_2O_2CO_3$ and crystalline anhydrous $La_2CO_5$, **characterized in that** the compound has a BET specific surface area of at least $10 m^2/g$ and wherein the compound, at an amount 3 times the stoichiometric amount needed to react completely with the phosphate, exhibits a phosphate binding of at least 40 wt%, preferably at least 60 wt% of the initial amount of phosphate in a stock solution at room temperature after 10 minutes, wherein the stock solution contains 13.75 g/l of anhydrous $Na_2HPO_4$ and 8.5 g/l HCl and has been adjusted to a pH of 3, and wherein the crystalline anhydrous $La_2O_2CO_3$ and crystalline anhydrous $La_2CO_5$ are in the form of particles with a porous structure, and wherein the compound is formed from individual crystals having a size from 20 nm to 10 $\mu$m.

2.  The compound according to claim 1, wherein the compound has a BET specific area of at least 20 $m^2/g$, preferably at least 35 $m^2/g$.

3.  The compound according to any one of claims 1-2, wherein the particles have a size from 1 $\mu$m to 1000 $\mu$m.

4.  The compound according to any one of claims 1-3, wherein the compound is crystalline anhydrous $La_2O_2CO_3$.

5.  The composition according to any one of claims 1-4, wherein the compound is crystalline anhydrous $La_2CO_5$.

6.  An orally ingestible form selected from the group consisting of a tablet, a capsule, and a gelcap wherein the orally ingestible form comprises the compound of any one of claims 1-5.

7.  The compound according to any one of claims 1-6 for use in the treatment of hyperphosphatemia.

8.  Use of a compound selected from the group consisting of crystalline anhydrous $La_2O_2CO_3$ and crystalline anhydrous $La_2CO_5$, with a BET specific surface area of at least $10m^2/g$, wherein the compound, at an amount 3 times the stoichiometric amount needed to react completely with the phosphate, exhibits a phosphate binding of at least 40 wt%, preferably at least 60 wt% of the initial amount of phosphate in a stock solution at room temperature after 10 minutes, wherein the stock solution contains 13.75 g/l of anhydrous $Na_2HPO_4$ and 8.5 g/l HCl and has been adjusted to a pH of 3, and wherein the crystalline anhydrous $La_2O_2CO_3$ and crystalline anhydrous $La_2CO_5$ are in the form of particles with a porous structure, and the compound is formed from individual crystals having a size from 20 nm to 10 $\mu$m, for the manufacture of a medicament for the treatment of hyperphosphatemia.

9.  The use according to claim 8, wherein the compound has a BET specific area of at least 20 $m^2/g$, preferably at least 35 $m^2/g$.

10. The use according to any one of claims 8-9, wherein the particles have a size from 1 $\mu$m to 1000 $\mu$m.

11. The use according to any one of claims 8-10, wherein the compound is crystalline anhydrous $La_2O_2CO_3$.

12. The use according to any one of claims 8-11, wherein the compound is crystalline anhydrous $La_2CO_5$.

13. A method of producing a crystalline anhydrous lanthanum oxycarbonate selected from the group consisting of crystalline anhydrous $La_2O_2CO_3$ and crystalline anhydrous $La_2CO_5$ and having a BET specific surface area of at least $10m^2/g$, and wherein the compound, at an amount 3 times the stoichiometric amount needed to react completely with the phosphate, exhibits a phosphate binding of at least 40 wt%, preferably at least 60 wt% of the initial amount of phosphate in a stock solution at room temperature after 10 minutes, wherein the stock solution contains 13.75 g/l of anhydrous $Na_2HPO_4$ and 8.5 g/l HCl and has been adjusted to a pH of 3, and wherein the crystalline anhydrous

$La_2O_2CO_3$ and crystalline anhydrous $La_2CO_5$ are in the form of particles with a porous structure, and wherein the compound is formed from individual crystals having a size from 20 nm to 10 $\mu$m, the method comprising:

a) reacting a lanthanum chloride in a solution with sodium carbonate at a temperature of 80 to 90°C to form a slurry or suspension, wherein a molar ratio of sodium carbonate to lanthanum chloride is 6:2, and wherein the sodium carbonate is added to the solution over a period of 2 hours;
b) curing the slurry or suspension for 2 hours at a temperature of 80°C;
c) recovering a precipitate from the slurry or suspension by filtering, washing and drying, wherein the drying is at a temperature ranging from 100 to 120°C; and
d) thermally treating the resulting precipitate at a temperature of 400 to 700°C for a period ranging from 1 to 48 hours to produce the anhydrous lanthanum oxycarbonate compound.

**Patentansprüche**

1. Verbindung, ausgewählt aus der Gruppe bestehend aus kristallinem wasserfreiem $La_2O_2CO_3$ und kristallinem wasserfreiem $La_2CO_5$, **dadurch gekennzeichnet, dass** die Verbindung eine spezifische BET-Oberfläche von mindestens 10 m2/g aufweist und wobei die Verbindung in einer Menge, die das Dreifache der stöchiometrischen Menge beträgt, die zur vollständigen Umsetzung mit dem Phosphat erforderlich ist, eine Phosphatbindung von mindestens 40 Gew.-%, vorzugsweise mindestens 60 Gew.-% der Ausgangsmenge an Phosphat in einer Stammlösung bei Raumtemperatur nach 10 Minuten aufweist, wobei die Stammlösung 13,75 g/l wasserfreies $Na_2HPO_4$ und 8,5 g/l HCl enthält und auf einen pH-Wert von 3 eingestellt worden ist, und wobei das kristalline wasserfreie $La_2O_2CO_3$ und das kristalline wasserfreie $La_2CO_5$ in Form von Partikeln mit einer porösen Struktur vorliegen und wobei die Verbindung aus einzelnen Kristallen mit einer Größe von 20 $\mu$m bis 10 $\mu$m gebildet ist.

2. Verbindung nach Anspruch 1, wobei die Verbindung eine spezifische BET-Fläche von mindestens 20 m$^2$/g aufweist, vorzugsweise von mindestens 35 m$^2$/g.

3. Verbindung nach einem der Ansprüche 1 bis 2, wobei die Partikel eine Größe von 1 $\mu$m bis 1000 $\mu$m aufweisen.

4. Verbindung nach einem der Ansprüche 1 bis 3, wobei die Verbindung kristallines wasserfreies $La_2O_2CO_3$ ist.

5. Verbindung nach einem der Ansprüche 1 bis 4, wobei die Verbindung kristallines wasserfreies $La_2CO_5$ ist.

6. Oral einnehmbare Form, ausgewählt aus der Gruppe bestehend aus einer Tablette, einer Kapsel und einer Gelkapsel, wobei die oral einnehmbare Form die Verbindung nach einem der Ansprüche 1 bis 5 umfasst.

7. Verbindung nach einem der Ansprüche 1 bis 6 zur Verwendung bei der Behandlung von Hyperphosphatämie.

8. Verwendung einer Verbindung, ausgewählt aus der Gruppe bestehend aus kristallinem wasserfreiem $La_2O_2CO_3$ und kristallinem wasserfreiem $La_2CO_5$ mit einer spezifischen BET-Oberfläche von mindestens 10 m$^2$/g, wobei die Verbindung in einer Menge, die das Dreifache der stöchiometrischen Menge beträgt, die zum vollständigen Umsetzen mit dem Phosphat erforderlich ist, eine Phosphatbindung von mindestens 40 Gew.-%, vorzugsweise mindestens 60 Gew.-% der Ausgangsmenge an Phosphat in einer Stammlösung bei Raumtemperatur nach 10 Minuten aufweist, wobei die Stammlösung 13,75 g/l wasserfreies $Na_2HPO_4$ und 8,5 g/l HCl enthält und auf einen pH-Wert von 3 eingestellt worden ist, und wobei das kristalline wasserfreie $La_2O_2CO_3$ und das kristalline wasserfreie $La_2CO_5$ in Form von Partikeln mit einer porösen Struktur vorliegen und die Verbindung aus einzelnen Kristallen mit einer Größe von 20 $\mu$m bis 10 $\mu$m gebildet ist, für die Herstellung eines Medikaments für die Behandlung von Hyperphosphatämie.

9. Verwendung nach Anspruch 8, wobei die Verbindung eine spezifische BET-Fläche von mindestens 20 m$^2$/g aufweist, vorzugsweise von mindestens 35 m$^2$/g.

10. Verwendung nach einem der Ansprüche 8 bis 9, wobei die Partikel eine Größe von 1 $\mu$m bis 1000 $\mu$m aufweisen.

11. Verwendung nach einem der Ansprüche 8 bis 10, wobei die Verbindung kristallines wasserfreies $La_2O_2CO_3$ ist.

12. Verwendung nach einem der Ansprüche 8 bis 11, wobei die Verbindung kristallines wasserfreies $La_2CO_5$ ist.

**13.** Verfahren zum Herstellen eines kristallinen wasserfreien Lanthan-Oxycarbonats, ausgewählt aus der Gruppe bestehend aus kristallinem wasserfreiem $La_2O_2CO_3$ und kristallinem wasserfreiem $La_2CO_5$ , **dadurch gekennzeichnet, dass** die Verbindung eine spezifische BET-Oberfläche von mindestens 10 m$^2$/g aufweist und wobei die Verbindung in einer Menge, die das Dreifache der stöchiometrischen Menge beträgt, die zur vollständigen Umsetzung mit dem Phosphat erforderlich ist, eine Phosphatbindung von mindestens 40 Gew.-%, vorzugsweise mindestens 60 Gew.-% der Ausgangsmenge an Phosphat in einer Stammlösung bei Raumtemperatur nach 10 Minuten aufweist, wobei die Stammlösung 13,75 g/l wasserfreies $Na_2HPO_4$ und 8,5 g/l HCl enthält und auf einen pH-Wert von 3 eingestellt worden ist, und wobei das kristalline wasserfreie $La_2O_2CO_3$ und das kristalline wasserfreie $La_2CO_5$ in Form von Partikeln mit einer porösen Struktur vorliegen und wobei die Verbindung aus einzelnen Kristallen mit einer Größe von 20 μm bis 10 μm gebildet ist, das Verfahren umfassend:

a) Umsetzen eines Lanthanchlorids in einer Lösung mit Natriumcarbonat bei einer Temperatur von 80 bis 90°C, um eine Aufschlämmung oder Suspension zu bilden, wobei ein Molverhältnis von Natriumcarbonat zu Lanthanchlorid 6:2 beträgt und wobei das Natriumcarbonat zu der Lösung über einen Zeitraum von 2 Stunden zugegeben wird;
b) Aushärten der Aufschlämmung oder Suspension für 2 Stunden bei einer Temperatur von 80°C;
c) Gewinnen eines Niederschlags aus der Aufschlämmung oder Suspension durch Filtrieren, Waschen und Trocknen, wobei das Trocknen bei einer Temperatur im Bereich von 100 bis 120°C erfolgt; und
d) thermisches Behandeln des resultierenden Niederschlags bei einer Temperatur von 400 bis 700°C für einen Zeitraum im Bereich von 1 bis 48 Stunden, um die wasserfreie Lanthan-Oxycarbonatverbindung herzustellen.

## Revendications

**1.** Composé sélectionné dans le groupe consistant en $La_2O_2CO_3$ cristallin anhydre et $La_2CO_5$ cristallin anhydre, **caractérisé en ce que** le composé présente une surface spécifique BET d'au moins 10 m$^2$/g et dans lequel le composé, à une quantité de 3 fois la quantité stoechiométrique nécessaire pour réagir complètement avec le phosphate, présente une liaison au phosphate d'au moins 40 % en poids, de préférence d'au moins 60 % en poids de la quantité initiale de phosphate dans une solution mère à température ambiante après 10 minutes, dans lequel la solution mère contient 13,75 g/l de $Na_2HPO_4$ anhydre et 8,5 g/l de HCl et a été ajustée à un pH de 3, et dans lequel le $La_2O_2CO_3$ cristallin anhydre et le $La_2CO_5$ cristallin anhydre sont sous la forme de particules à structure poreuse, et dans lequel le composé est formé à partir de cristaux individuels présentant une taille de 20 μm à 10 μm.

**2.** Composé selon la revendication 1, dans lequel le composé présente une surface spécifique BET d'au moins 20 m$^2$/g, de préférence d'au moins 35 m$^2$/g.

**3.** Composé selon l'une quelconque des revendications 1-2, dans lequel les particules présentent une taille de 1 μm à 1000 μm.

**4.** Composé selon l'une quelconque des revendications 1-3, dans lequel le composé est $La_2O_2CO_3$ cristallin anhydre.

**5.** Composé selon l'une quelconque des revendications 1-4, dans lequel le composé est $La_2CO_5$ cristallin anhydre.

**6.** Forme ingérable par voie orale sélectionnée dans le groupe consistant en un comprimé, une capsule, et une gélule dans laquelle la forme ingérable par voie orale comprend le composé selon l'une quelconque des revendications 1-5.

**7.** Composé selon l'une quelconque des revendications 1-6 pour une utilisation dans le traitement de l'hyperphosphatémie.

**8.** Utilisation d'un composé sélectionné dans le groupe consistant en $La_2O_2CO_3$ cristallin anhydre et $La_2CO_5$ cristallin anhydre, présentant une surface spécifique BET d'au moins 10 m$^2$/g, dans laquelle le composé, à une quantité de 3 fois la quantité stoechiométrique nécessaire pour réagir complètement avec le phosphate, présente une liaison au phosphate d'au moins 40 % en poids, de préférence d'au moins 60 % en poids de la quantité initiale de phosphate dans une solution mère à température ambiante après 10 minutes, dans laquelle la solution mère contient 13,75 g/l de $Na_2HPO_4$ anhydre et 8,5 g/l de HCl et a été ajustée à un pH de 3, et dans laquelle le $La_2O_2CO_3$ cristallin anhydre et le $La_2CO_5$ cristallin anhydre sont sous la forme de particules à structure poreuse, et le composé est formé à partir de cristaux individuels présentant une taille de 20 nm à 10 μm, pour la fabrication d'un médicament pour le traitement de l'hyperphosphatémie.

9. Utilisation selon la revendication 8, dans laquelle le composé présente une surface spécifique BET d'au moins 20 $m^2$/g, de préférence d'au moins 35 $m^2$/g.

10. Utilisation selon l'une quelconque des revendications 8-9, dans laquelle les particules présentent une taille de 1 $\mu$m à 1000 $\mu$m.

11. Utilisation selon l'une quelconque des revendications 8-10, dans laquelle le composé est $La_2O_2CO_3$ cristallin anhydre.

12. Utilisation selon l'une quelconque des revendications 8-11, dans laquelle le composé est $La_2CO_5$ cristallin anhydre.

13. Procédé de production d'un oxycarbonate de lanthane cristallin anhydre sélectionné dans le groupe consistant en $La_2O_2CO_3$ cristallin anhydre et $La_2CO_5$ cristallin anhydre et présentant une surface spécifique BET d'au moins 10 $m^2$/g, et dans lequel le composé, à une quantité de 3 fois la quantité stoechiométrique nécessaire pour réagir complètement avec le phosphate, présente une liaison au phosphate d'au moins 40 % en poids, de préférence d'au moins 60 % en poids de la quantité initiale de phosphate dans une solution mère à température ambiante après 10 minutes, dans lequel la solution mère contient 13,75 g/l de $Na_2HPO_4$ anhydre et 8,5 g/l de HCl et a été ajustée à un pH de 3, et dans lequel le $La_2O_2CO_3$ cristallin anhydre et le $La_2CO_5$ cristallin anhydre sont sous la forme de particules à structure poreuse, et dans lequel le composé est formé à partir de cristaux individuels présentant une taille de 20 nm à 10 $\mu$m, le procédé comprenant :

a) la réaction d'un chlorure de lanthane en solution avec du carbonate de sodium à une température de 80 à 90 °C pour former une bouillie ou une suspension, dans lequel un rapport molaire du carbonate de sodium sur le chlorure de lanthane est de 6 : 2, et dans lequel le carbonate de sodium est ajouté à la solution sur une période de 2 heures ;
b) le durcissement de la bouillie ou de la suspension pendant 2 heures à une température de 80 °C ;
c) la récupération d'un précipité à partir de la bouillie ou de la suspension par filtration, lavage et séchage, dans lequel le séchage est à une température allant de 100 à 120 °C ; et
d) le traitement thermique du précipité résultant à une température de 400 à 700 °C pendant une période allant de 1 à 48 heures pour produire le composé d'oxycarbonate de lanthane anhydre.

**FIG. 1**

Solution of
lanthanum chloride

↓

```
┌─────────────────────┐
│         20          │
│     EVAPORATION     │
└─────────────────────┘
```

↓

```
┌─────────────────────┐
│         30          │
│     CALCINATION     │
└─────────────────────┘
```

↓

```
┌─────────────────────┐
│         40          │
│      WASHING        │
└─────────────────────┘
```

Calcined
lanthanum
oxychloride
product

FIG. 2

Solution of
titanium chloride
or oxychloride

104
Chemical Control
Agents

110
MIXING

120
EVAPORATION

130
FIRST
CALCINATION

Calcined
product

140
WASHING

Solution of
inorganic
compound

150
MIXING

160
EVAPORATION

170
SECOND
CALCINATION

Final product:
Coated porous
structure

180
WASHING

# FIG. 3

Solution of
lanthanum acetate

↓

```
┌─────────────────────┐
│        220          │
│   EVAPORATION       │
└─────────────────────┘
```

↓

```
┌─────────────────────┐
│        230          │
│   CALCINATION       │
└─────────────────────┘
```

↓

```
┌─────────────────────┐
│        240          │
│     WASHING         │
└─────────────────────┘
```

↓

Calcined
lanthanum
oxycarbonate
product

FIG. 4

FIG. 5

FIG. 6

FIG. 7

FIG. 8

FIG. 9

## FIG. 10

$H_2O$   $La_2O_3$   HCl

LaCl$_3$ solution

LaCl$_3$ solution, heat to 80° C

Aq. Na$_2$CO$_3$ solution addition over 2 hrs

La$_2$O(CO$_3$)$_2$·xH$_2$O Formation at 80° C

La$_2$O(CO$_3$)$_2$·xH$_2$O Wash and filter (remove salts)

La$_2$O(CO$_3$)$_2$·xH$_2$O Dry at 105°C for 2 hours.

x, the number of water molecules in the compound, is larger or equal to 2 and smaller or equal to 4.

# FIG. 11

H₂O      La₂O₃      HCl

```
              ┌─────────────────────────┐
              │      LaCl₃ solution      │
              └─────────────────────────┘
                           │
                           ▼
              ┌─────────────────────────┐
              │  LaCl₃ solution, heat to │
              │          80°C            │
              └─────────────────────────┘
                           │
Aq. Na₂CO₃ solution        │
addition over 2 hrs ──►    ▼
              ┌─────────────────────────┐
              │   La₂O(CO₃)₂•xH₂O        │
              │   Formation at 80°C      │
              └─────────────────────────┘
                           │
                           ▼
              ┌─────────────────────────┐
              │   La₂O(CO₃)₂•xH₂O        │
              │   Wash and filter        │
              │   (remove salts)         │
              └─────────────────────────┘
                           │
                           ▼
              ┌─────────────────────────┐
              │   La₂O(CO₃)₂•4H₂O        │
              │   Dry at 105°C for       │
              │   2 hours.               │
              └─────────────────────────┘
                           │
                           ▼
              ┌─────────────────────────┐
              │  La₂O₂CO₃ or La₂CO₅      │
              │  formation               │
              │  Calcine at 500°C or 600°C │
              └─────────────────────────┘
```

**FIG. 12**

EP 2 194 028 B1

FIG. 13

32

## FIG. 14

FIG. 15

## FIG. 16

## FIG. 17

FIG. 18

Altair 3.0kV 7.4mm x50.0k SE(U) 7/22/02                    1.00um

FIG. 19

**FIG. 20**

## FIG. 21

ALTAIR 3.0kV 6.4mm X60.0k SE(U) 8/6/02    500nm

FIG. 22

File: L71DC600-7h-B(1492-3802), ID: L80DC600°C/65h 8-5-02
Date: 08/06/02 08:10  Step : 0.020° Cnt Time: 0.600 Sec.
Range: 2.00 - 80.00 (Deg) Cont. Scan Rate : 2.00 Deg/min.

23-0320 : LANTHANUM OXIDE CARBONATE/LA2 C O5
23-0322 : LANTHANUM OXIDE CARBONATE/LA2 C O5

23-0322    LANTHANUM OXIDE CARBONATE

FIG. 23

**FIG. 24**

length of scale bar = 2 micron

**FIG. 25**

Altair 3.0kV 4.5mm x300k SE(U) 4/18/02   1.00μm

FIG. 26

Altair 3.0kV 4.3mm x35.0k SE(U) 4/18/02    1.00um

FIG. 27

Altair 3.0kV 4.6mm ×10.0k SE(U) 4/18/02    5.00um

Fig.28

FIG. 29

FIG. 30

FIG. 31

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4497785 A
- US 4929787 A
- US 4919902 A
- JP 61004529 A
- US 5968976 A
- US 6322895 B
- US 5782792 A

**Non-patent literature cited in the description**

- **LIU S et al.** Synthesis and structure characterization of hydrated lanthanum carbonate. *HUANAN LIGONG DAXUE XUEBAO - JOURNAL OF SOUTH CHINA UNIVERSITY OF TECHNOLOGY, GUANGZHOU,* 01 October 1997, vol. 25 (10), ISSN 1000-565X, 23-26